(19)

Europäisches
Patentamt
European
Patent Office
Office européen
des brevets

(11) **EP 2 412 813 A1**

(12) **EUROPEAN PATENT APPLICATION**

(43) Date of publication:
**01.02.2012 Bulletin 2012/05**

(51) Int Cl.:
*C12N 15/63* (2006.01)          *C12N 15/75* (2006.01)

(21) Application number: **10171252.9**

(22) Date of filing: **29.07.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Lonza AG
2930 Visp VS (CH)**

(72) Inventors:
• **Wenzel, Marian
73272 Neidlingen (DE)**

• **Altenbuchner, Josef
71154 Nufingen (DE)**
• **Kiziak, Christoph
3939 Visp (CH)**

(74) Representative: **Ahrens, Gabriele et al
Patentanwälte Einsel & Kollegen
Jasperallee 1A
38102 Braunschweig (DE)**

(54) **Regulation of inducible promoters**

(57)     The present invention relates to the production of heterologous polypeptides in a recombinant bacterial host cell, wherein the bacterial host cell is rendered inable to deactivate the promoter controlling the expression of the heterologous polypeptide in the absence of an inducer.

**Description**

The present invention relates to the production of heterologous polypeptides in a recombinant bacterial host cell. In particular, the present invention relates to the regulation of the expression of a heterologous nucleic acid sequence encoding for the polypeptide by the use of a promoter inducible by a specific substrate (inducer). Most particularly, the present invention relates to the regulation of the expression of heterologous polypeptide in a bacterial host cell wherein the bacterial host cell is rendered inable to deactivate the promoter controlling the expression of the heterologous polypeptide in the absence of inducer.

**Background of the Invention**

An important aspect in heterologous polypeptide production in recombinant microorganism is the selection of the promoter used for controlling the expression of the heterologous nucleic acid sequences which encodes the target polypeptide.

A suitable promoter should be strong. That is, produce the respective mRNA in a high rate allowing production of the polypeptide in high amount. Further, the promoter should be readily to be regulated and start the production of the heterologous polypeptide only upon induction.

However, there is the problem that typically the inducing substrate is a nutrient for the microorganism and is consumed by the microorganism. However, when the medium used for cultivating the microorganism runs out of the inducing substrate, the microorganism deactivates the promoter and, thus, expression of the target polypeptide stops. For avoiding unwanted stop of gene expression, the inducer must be added in high amounts and/or continuously supplemented. The need of high amounts of inducer raises the costs of the fermentation process. Further, use of efficient promoters which, however, require expensive inducers is restricted.

Consequently, for the recombinant polypeptide production host cells are desired wherein the activity of the promoter controlling the expression of the heterologous polypeptide is independent from the presence of the inducer but wherein expression of the heterologous polypeptide can be nevertheless tightly regulated. WO 2006/133210 A2 relates to a method for producing recombinant peptides in a bacterial host cell utilizing a mannitol, arabitol, glucitol or glycerol-inducible promoter, wherein the bacterial host cell has been rendered incapable of degrading or metabolizing the inducer. According to said method a gene or genes encoding for enzymes required for metabolizing the inducer is genetically altered or deleted in the genome so that the cell cannot express, from its genome, a functional enzyme necessary for metabolizing or degrading the inducer. In order to ensure uptake of the inducer genes related to the transport of the inducer into the cell, are unaffected.

This method, however, requires nevertheless addition of inducer for catalyzing activation of the respective promoter controlling expression of the target polypeptide. Further, accumulation of inducer in the cell can negatively affect development of the cell.

Many bacteria are able to utilize different carbon sources. If provided with a mixture of carbon sources the carbon source that allows the most rapid growth (primary carbon source) is selected. Simultaneously the functions involved in the utilization of secondary carbon sources are repressed by a phenomenon called carbon catabolite repression (CCR).

Besides CCR the specific catabolic genes involved in the utilization of a less preferred secondary carbon source are only expressed in the presence of said secondary carbon source. Consequently, expression of genes involved in catabolismn of a secondary carbon source depends on the presence of said secondary carbon source (induction) and the absence of a primary carbon source (catabolite repression).

**Summary of the Invention**

The present invention makes use of these regulation mechanisms of carbon catabolism by providing a bacterial host cell wherein the promoter regulating the expression of the genes involved in the metabolism of a secondary carbon source is not deactivated in the absence of its corresponding carbon source, and wherein the promoter is solely under control of carbon catabolite repression.

According to the present invention, the promoter used is a promoter which regulates the utilization of a secondary carbon source of the bacterial host cell.

In the presence of a primary carbon source the promoter is repressed by CCR. When the medium used to cultivate the recombinant bacterial host cell runs out of primary carbon source or the concentration of primary carbon source decreases below a level required for CCR, CCR of the promoter is rendered inoperative and the promoter automatically starts expression of the genes controlled by said promoter.

The present invention provides for a recombinant bacterial host cell wherein the recombinant bacterial host cell is capable of utilizing more than one carbon source, wherein carbon catabolism of these carbon sources of the bacterial host cell is subjected to the phosphoenolpyruvate: carbohydrate phosphotransferase system (PTS) and CCR, wherein the bacterial host cell is genetically altered to prevent deactivation of the transcriptional regulator protein of a carbon source inducible promoter in the absence of said secondary carbon source, but is under control of CCR, wherein the carbon source is a secondary carbon source for the bacterial host cell.

According to a further aspect of the present invention the recombinant bacterial host cell of the present invention is transformed with a vector comprising a heterologous nucleic acid sequence encoding a polypeptide operably linked to a promoter inducible by a secondary carbon source, wherein the promoter of the vector is controlled by the transcriptional regulator protein for which the bacterial host cell has been genetically altered to be incapable of deactivation in absence of the specific corresponding carbon source.

Furthermore, the present invention provides for a process for preparing heterologous polypeptides by culturing the recombinant bacterial host cell of the present invention transformed with a vector which comprises the nucleic acid sequence encoding for the polypeptide.

Further, the present invention relates to the use of a recombinant bacterial host cell according to the present invention in the production of heterologous polypeptides.

Other objects and advantages will become apparent to those skilled in the art from review of the following detailed description with reference to the accompanying illustrative figures and the attached claims.

According to the embodiment referred to above induction of the expression of a target polypeptide is independent of the presence of an inducing carbon source for the promoter. Thus, this embodiment is particularly advantageous in that no inducing carbon source is required.

However, it would be also helpful from an economical point of view, if the amount of carbon source necessary for inducing the promoter can be reduced.

Thus, according to an alternative solution the present invention relates to a method for producing a target polypeptide by expression of a nucleotide sequence encoding for said target polypeptide, wherein the expression is under control of a carbon source inducible promoter, wherein the process of catabolism of the inducing carbon source by the bacterial host cell, which shall be transformed with a vector carrying the promoter and the nucleotide sequence of a target polypeptide, is interrupted or at least retarded.

According to the present invention this alternative is achieved by eliminating or genetically altering in the genome of the bacterial host cell the nucleotide sequence encoding for the inducing carbon source specific isomerase, which converts the inducing carbon source, once transported into the cell, to fructose-6-phosphate. Isomerisation to fructose-6-phosphate is the first step in catabolism of a carbon source once the carbon source has been transported into the cell. Interrupting or retarding the isomerisation of the carbon source means that the carbon source is available for induction for a prolonged period of time. Consequently, the amount of inducing carbon source can be reduced.

According to an example this alternative relates to a mannose inducible promoter, and a bacterial host cell for such promoter wherein in the genome of the bacterial host cell the gene encoding the mannose-6-phosphate isomerase, also referred to ManA, has been eliminated or genetically altered so that isomerisation of mannose, once transported into the cell, is not possible or at least retarded.

**Brief Description of the Figures**

It is shown in

| | |
|---|---|
| **Figure 1** | schematically the structure of the mannose operon with the arrangement and orientation of the respective genes and the promoters and activation thereof by ManR indicated by arrows; |
| **Figure 2** | a flow chart of the mannose catabolism with the transport into the cell, phosphorylation of mannose to mannose-6-phosphate during transport and conversion to fructose-6-phosphate; |
| **Figure 3** | schematically the structure of the ManR activator protein with the various domains and potential phosphorylation sites; |
| **Figure 4** | the nucleic acid sequence of the promoter region of *B.subtilis* comprising *manR* promoter; |
| **Figure 5** | the nucleic acid sequence from *B.subtilis* used in the the promoter-probe vector pSUN272.1 for studying inducibllility and catabolite repression of the *manP* promoter by mannose and glucose and determination of the ManR binding site; |
| **Figure 6** | mechanism of catabolite repression and activation in B. *subtilis* via CcpA; |

| Figure 7 | the plasmid map of the expression vector pSUN279.2; |
| Figure 8 | the β-galactosidase activities of *B.subtilis* 3NA containing the plasmids pSUN279.2, pSUN284.1 and pSUN291, respectively; |
| Figure 9 | the β-galactosidase activities of *B.subtilis* 3NA containing the plasmid pSUN 284.1 as well as further plasmids containing fragments of different lengths of the nucleic acid sequence shown in figure 5; |
| Figure 10 | the β-galactosidase activities of *B.subtilis* 3NA comprising the vectors pSUN291, pSUN385.2 and pSUN386.9 with the nucleic acid sequences as shown in figure 4; |
| Figure 11 | the plasmid map of expression vector pMW168.1; |
| Figure 12 | the plasmid map of insertion vector pSUN356.7 (Δ*manP*); |
| Figure 13 | the plasmid map of insertion vector pMW343.1 *(ΔmanA)*; |
| Figures 14 and 15 | a diagram showing logarithmically the dry biomass concentration plotted over the process period of the fermentation of *B.subtilis* TQ356/pMW168.1 (Δ*manP*-mutant) and a diagram with the fluorescence signal (RFU) plotted over the process period; |
| Figures 16 and 17 | a diagram showing logarithmically the dry biomass concentration plotted over the process period of fermentation of *B.subtilis* MW343/pMW168.1 (Δ*manA*-mutant) and a diagram with the fluorescence signal (RFU) plotted over the process period of fermentation; |
| Figure 18 | a diagram with the fluorescence signal (RFU) plotted over the process period of a further fermentation of *B.subtilis* MW343/pMW168.1 (Δ*manA*-mutant). |

**Detailed Description of the Invention**

As used herein, the following definitions are supplied in order to facilitate the understanding of the present invention.

"Enzyme EII", "EII" or "transporter" refer to a carbon source specific permease of the phosphoenolpyruvate: carbohydrate phosphotransferase system (PTS), which catalyzes the transport and concomitant phosphorylation of the carbon source.

The PTS comprises a variety of EIIs, each specific for a carbon source, for example a sugar.

EIIs are complexes usually consisting of three domains A, B and C, and sometimes a forth domain D, wherein EIIA and EIIB participates in phosphorylation of the corresponding carbon source, and the membrane bound EIIC (EIID, if present) mediates passage of the specific carbon source into the cell.

In the absence of the specific carbon source the corresponding EIIA and, in some cases, EIIB deactivate the respective carbon source specific transcriptional regulator protein by transfer of phosphoryl groups to corresponding phosphorylation sites present in the transcriptional regulator protein, referred to - depending on the phosphorylating EII - EIIA and EIIB domain, respectively.

"Transcriptional regulator protein" or "regulator" positively regulates (i.e. activates) the catabolic operon(s) of the specific carbon source. The transcriptional regulator proteins usually contain two conserved regulatory domains that can be phosphorylated (PTS regulatory domains, PRDs). Further, some transcriptional regulator proteins in addition contain further phosphorylation sites referred to EIIA and EIIB. Depending on the transcriptional regulator protein the transcriptional regulator protein is deactivated by phosphoryl group transfer from enzyme II to one or more of the above phosphoryl binding sites in the EIIA and EIIB and/or PRDI domain, and activated by phosphoryl group transfer from histidine protein (HPr) to the PRDII domain. The various carbon source specific transcriptional regulator proteins of the PTS can be activators or antiterminators.

"Promoter" as used herein refers to a nucleic acid sequence that regulates expression. A "promoter region" is a regulatory region capable of binding RNA polymerase in a cell and initiating transcription of a downstream (3'direction) coding sequence. Within the promoter region will be found protein binding domains (consensus sequences) responsible for the binding of RNA polymerase such as the -35 box and the -10 box (Pribnow box). Further, the promoter region may comprise the transcription start site and binding sites for their specific transcriptional regulator protein.

With "variants" or "variants of a sequence" is meant a nucleic acid sequence that varies from the reference sequence by conservative nucleic acid substitutions, whereby one or more nucleic acids are substituted by another with same characteristics. Variants encompass as well degenerated sequences, sequences with deletions and insertions, as long as such modified sequences exhibit the same function (functionally equivalent) as the reference sequence.

A "vector expressible in a host" or "expression vector" is a polynucleic acid construct, generated recombinantly or synthetically, with a series of specified polynucleic acid elements that permit transcription of particular nucleic acid sequence in a host cell. Typically, this vector includes a transcriptional unit comprising a particular nucleic acid sequence to be transcribed operably linked to a promoter. A vector expressible in a host can be for example an autonomously or self-replicating plasmid, a cosmid, a phage, a virus or a retro-virus.

The terms "transformation", "transformed" or "introducing a nucleic acid into a host cell" denote any process wherein an extracellular nucleic acid like a vector, with or without accompanying material, enters a host cell.

Transformation of appropriate host cells with, for example, an expression vector can be accomplished by well known methods such as microinjection, electroporation, particle bombardement or by chemical methods such as Calcium phosphate-mediated transformation and by natural transformation systems, described, for example, in Maniatise et al., Molecular Cloning A laboratory Manual, Cold Spring Harbor Laboratory (1982) or in Ausubel et al., Current protocols in molecular biology, John Wiley and Sohns (1984).

"Heterologous nucleic acid sequence" or "nucleic acid sequence heterologous to a host" means a nucleic acid sequence which encodes, for example, an expression product such as a polypeptide that is foreign to the host "heterologous expression" or "heterologous product" i. e. a nucleic acid sequence originating from a donor different from the host or a chemically synthesized nucleic acid sequence with encodes, for example, an expression product such as a polypeptide that is foreign the host. In case the host is a particular prokaryotic species, the heterologous nucleic acid sequence is preferably originated from a different genus of family, more preferred from a different order or class, in particular from a different phylum (division) at most particular from a different domain (empire) of organisms.

The heterologous nucleic acid sequence originating from a donor different from the host can be modified, before it is introduced into the host cell, by mutations, insertions, deletions or substitutions of single nucleic acids or a part of the heterologous nucleic acid sequence as long as such modified sequences exhibit the same function (functionally equivalent) as a reference sequence. A heterologous nucleic acid sequence as referred herein encompasses as well nucleic sequences originating from a different domain (empire) of organisms such as from eukaryotes (of eukaryotic origin) such as, for example, human men antibodies which have been used in phage display libraries and of which single nucleic acids or a part of the nucleic acid sequences have been modified according to the "codon usage" of a prokaryotic host.

"Heterologous polypeptide" or "target polypeptide" within the meaning of the present invention can be a heterologous protein of human, mammalian or prokaryotic origin. Other proteins are antigens such as glycoproteins and carbohydrates from microbial pathogens, both viral and antibacterial, and from tumors. Other heterologous polypeptides are enzymes like chymosin, proteases, polymerases, dehydrogenases, nucleases, glucanases, oxidases, alpha-amylase, oxidoreductases, lipases, amidases, nitril hydratases, esterases or nitrilases.

"Carbon source" refers to a carbon source, typically a carbohydrate, which can be taken up and metabolized by a bacterial cell and is subject to PTS and carbon catabolite repression (CCR), typical examples for carbohydrates are sugars and sugar derivates.

Many bacteria can utilize more than one carbohydrate as a source of carbon and energy. By using specific extracellular enzymes bacteria such as Bacilli are capable of degrading several polysaccharides that are present in large amount in plant biomass. The resulting oligo-,di-, or monosaccharides are transported into the cell and further processed. Usually, the catabolic enzymes involved in the metabolism or degradation of the saccherides are synthesized only when the specific substrate is present in the culture medium and preferred carbon and energy sources are absent. A preferred carbohydrate transport pathway for transporting carbohydrates through the membrane of the cell of bacteria is the PTS.

In the PTS the transport of the carbohydrate through the membrane and subsequent phosphorylation is mediated by an enzyme specific for said carbohydrate referred to enzyme II (EII). Since EII mediates the transport of its corresponding carbon source into the cell EII is also referred to "transporter".

In the presence of a mixture of carbohydrates cells selectively take up the carbon source that provide them with the most energy and growth advantage (primary carbon source). Simultaneously, they repress the various functions involved in the catabolism and uptake of the less preferred carbon sources (secondary carbon source)

Typically, a primary carbon source for most bacteria is glucose and depending on the bacterium various other sugars and sugar derivates being used as secondary carbon sources. However, a primary carbon source can also be another compound. E.g. in case of pseudomonads a primary carbon can be an aromatic compound.

Secondary carbon sources include e.g. mannose, lactose and melibiose without being restricted to these.

In the PTS the various catabolic genes involved in the metabolism of specific carbon source are controlled by transcriptional regulator proteins. These transcriptional regulator proteins can act as antiterminators or transcription activator and are only active in the presence of a specific carbon source (inducer). It has been found that EII has a negative (deactivating) regulation effect to its corresponding transcriptional regulator protein by transferring phosphoryl groups to a specific binding site present in the transcriptional regulator protein.

In the absence of a primary carbon source and presence of the promoter specific inducing carbon source the promoter is activated by its corresponding transcriptional regulator protein and genes under control of this promoter are expressed. In the absence of the inducing carbon source the transcriptional regulator protein regulating the promoter is deactivated by phosphoryl group transfer from its EII to the respective binding site on the transcriptional regulator protein, thereby deactivating the promoter and stopping expression of the genes under control of said promoter.

Otherwise, in the presence of a preferred primary carbon source - irrespectively whether or not less preferred secondary carbon sources are present - expression of the catabolic genes of said secondary carbon sources are repressed by CCR.

Generally, the present invention is based on the inhibition of regulation of the carbon source specific transcriptional regulator protein in the absence of said specific carbon source. In particular, the present invention is based on preventing repression or deactivation by phosphoryl group transfer via the corresponding EII to the transcriptional regulator protein.

If repression of a carbon source specific transcriptional regulator protein is prevented, a promoter for which said transcriptional regulator protein is an activator is active irrespective of the presence of a carbon source being an inducer for said promoter. Consequently, no inducing carbon source is necessary for expression of a gene under control of such a promoter and, further, the gene is continuously expressed.

In view of the above the present invention makes use of a promoter inducible by a secondary carbon source wherein the promoter is controlled by PTS on one side and by CCR on the other side.

Accordingly the present invention seeks to prevent deactivation of a carbon source inducible promoter used as promoter in the expression of a target polypeptide by preventing deactivation of the transcriptional regulator protein specific for said promoter.

According to a first approach this goal is achieved by interrupting phosphorylation of the transcriptional regulator protein by its specific EII by rendering at least one binding site of the transcriptional regulator protein for a phosphoryl group transferred by EII unable to bind the phosphoryl group.

To this, in the genome of the bacterial host cell, the gene encoding for the transcriptional regulator protein can be genetically manipulated so that the gene expresses a transcriptional regulator protein which is incapable of binding a phosphoryl group transferred from EII.

According to a second approach phosphorylation is interrupted by deleting, in the genome of the bacterial host cell, the gene encoding for EII, i. e. the enzyme regulating activity of the transcriptional regulator protein.

According to the present invention expression of a heterologous polypeptide is put under control of a promoter which is specific for the transcriptional regulator protein referred to above, for which deactivation by phosphoryl group transfer via EII is prevented by genetical alteration of the bacterial host cell.

The present invention provides for an advantageous system for producing heterologous polypeptides by fermentation of the recombinant bacterial host cell of the present invention transformed with a vector comprising the heterologous nucleic acid encoding for said polypeptide operably linked to a carbon source inducible promoter, wherein the promoter is active even when no inducing carbon source is present in the fermentation medium. Since the promoter controlling the expression of the nucleic acid sequence encoding for the target polypeptide is still under control of carbon catabolite repression no expression takes place in the presence of a primary carbon source such as glucose, but induction is achieved automatically when the system runs out of the primary carbon source (auto-induction). Since activity of the promoter controlling expression of the heterologous polypeptide is independent from the presence of an inducing carbon source, in the fermentation medium induction of expression of the target polypeptide is achieved without the need of an inducing carbon source.

The present invention is advantageous in that the recombinant bacterial host cells can be grown to high cell density in the presence of their primary carbon source, and as soon as a desired cell density is achieved, production of the target polypeptides starts automatically on release of carbon catabolite repression of the promoter controlling expression.

Suitable bacterial host cells for the present invention are those which can utilize more than one carbon source wherein utilization of the different carbon sources is subject to carbon catabolite repression and wherein the transport of the carbon source through the membrane and phosphorylation thereof is subject to the phosphoenolpyruvate: carbohydrate phosphotransferase system.

Bacterial host cells suitable for the present invention can be Gram-positive or Gram-negative bacteria. Preferred examples are those belonging to the phylum Firmicutes, and, in particular, those belonging to the class Bacilli. Specific examples are those of genus *Bacillus* such as *B.subtilis, B.amyloliquifaciens, B.licheniformis, B.natto, B.megaterium,* etc., other preferred examples include i.a. Streptococcus, Staphylococcus, Lactobacillus, Escherichia or other member of the Enterobacteria, without being restricted to.

Typically, Firmicutes are gram-positive and have low GC-content. By "low GC-content" is meant that less than 52 % of the base pairs in the bacterial genome are GC pairs. For example, gram-positive bacteria such as those of *Bacillus* and *Clostridium* contain 40 % or less GC pairs in the genome.

Further suitable bacterial host cells are enteric bacteria such as those belonging to the order Enterobacteriales. Examples of such enteric bacteria are those being gram-negative such as of genus *Escherichia.* Specific examples are strains of *E.coli* such as TG1, W3110, DH1, XL1-Blue and Origami.

*E.coli* and enteric bacteria contain about 50 % GC content in the genome and are therefore low GC content organisms.

Gram-positive and Gram-negative organisms are determined according to the well known gram-staining procedure. Gram-positive organisms are those that assume a violet color under standard gram-staining. Gram-negative organisms incorporate the counter stain rather than the primary gram-stain.

There are different mechanisms of CCR in Firmicutes and enteric bacteria which have been intensively studied in *Bacillus subtilis* and *E.coli* as model organisms (reference is made for example to J. Stülke et al., "Regulation of carbon catabolism in Bacillus species", Annu. Rev. Microbiol. (2000) 54: 849 - 880; Görke B. et Stülke J., "Carbon catabolite repression in bacteria: many ways to make the most out of nutrients", Nat. Rev. Microbiol. (2008) 6: 613 - 624; Gosset G. et al., Transcriptome analysis of Crp-dependent catabolite control of gene expression in Escherichia coli", J. Bacteriol., (2004) 186: 3516 - 3524, Martinez-Antonio A. et al., Identifying global regulators in transcriptional regulatory networks in bacteria" Curr. Opin. Microbiol. (2003) 6: 482 - 489). Though the overall mechanism of CCR is different the result is the same, namely that in the presence of a primary carbon source the various catabolic operons involved in uptake and phosphorylation of secondary carbon sources are repressed.

The recombinant bacterial host cell of the present invention is genetically altered to prevent repression of a carbon source inducible promoter controlling the expression of a heterologous nucleic acid sequence by inhibiting deactivation of the promoter specific transcriptional regulator protein in absence of said inducing carbon source.

Thus, in the recombinant bacterial host cell of the present invention the carbon source inducible promoter controlling the expression of the heterologous nucleic acid sequence is subject to carbon catabolite repression only. In the presence of a primary carbon source expression of the heterologous polypeptide is repressed by CCR. According to the present invention the carbon source inducible promoter is in its active state irrespective of the presence of the inducing carbon source unless carbon catabolite repression is stimulated by a more preferred primary carbon source.

The present invention, thus, provides for the production of heterologous polypeptide without the need of an inducer for inducing the promoter controlling the gene(s) encoding the polypeptide.

Generally, promoters suitable for the present invention are those which are subject to PTS and CCR in bacterial cells. In particular, the respective transcriptional regulator proteins are activators or antiterminators for the catabolic operon under control of said promoters. Such promoters are known in the art. Examples of promoters suitable for the present invention are given in the table below by reference to the respective operon:

| Operon | Regulator a) | Type b) | Inducer | Organism |
|---|---|---|---|---|
| *sacPA* | SacT | AT | sucrose | *B.subtilis* |
| *sacB* | SacY | AT | sucrose | *B.subtilis* |
| *bgl PH* | LicT | AT | β-glucosides | *B.subtilis* |
| *licBCAH* | LicR | A | oligo-β-glucosides | *B.subtilis* |
| *levDEFG sacL* | LevR | A | fructose | *B.subtilis* |
| *mtlAD* | MtlR | A | mannitol | *B.subtilis* |
| *manPA-yjdF* | ManR | A | mannose | *B.subtilis* |
| *manR* | ManR | A | mannose | *B.subtilis* |
| *bglFB bglG* | BglG | AT | β-glucosides | *E. coli* |
| *lacTEGF* | LacT | AT | lactose | *L. casei* |
| a: transcriptional regulator protein b: A: activator AT: antiterminator | | | | |

In the following the present invention is explained in more detail by reference to the mannose operon of *B.subtilis.*

*B.subtilis* can use a plurality of different mono- or di-saccharides as carbon source such as glucose, maltose, sucrose, mannose, mannitol, and fructose. These saccharides are taken up by the PTS system. Transport into the cell and phosphorylation are mediated by the enzyme EII specific for the respective saccharide.

As in many bacteria the preferred carbon source of *B.subtilis* is glucose. In presence of glucose uptake of any of the other saccharide subject to PTS is repressed by CCR.

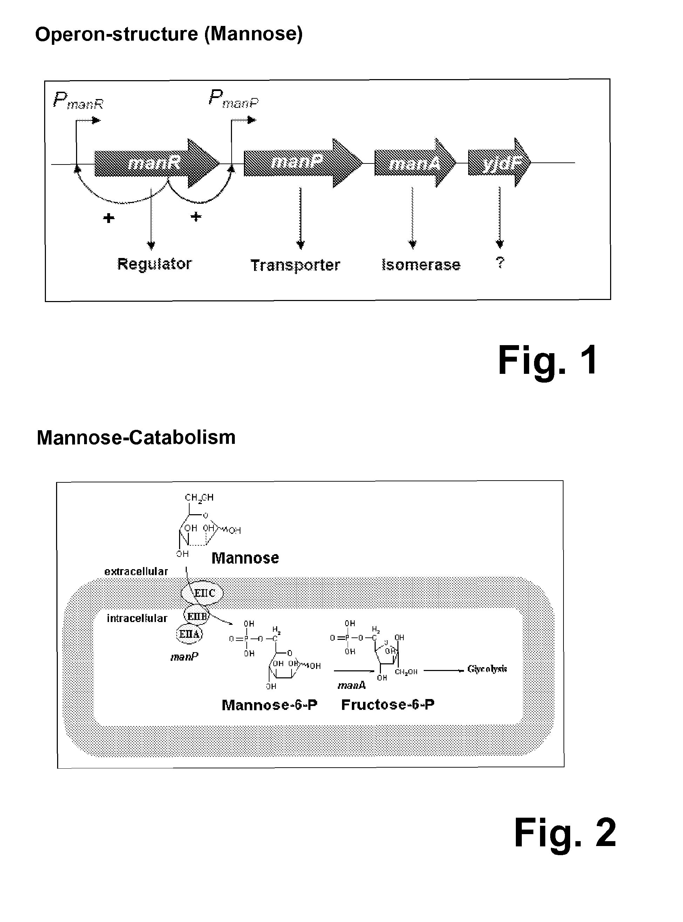

The operon structure of mannose is shown in figure 1 and the transport of mannose into the cell and catabolism thereof in figure 2. The mannose operon of *B.subtilis* comprises three catabolic genes (Kunst F. N. et al., "The complete genome sequence of gram-positive bacterium B.subtilis", Nature (1997) 390: 249 - 256).

The first gene, *manP,* encodes the mannose specific EII enzyme referred to ManP. ManP effects the transport of mannose through the membrane and, simultaneously, phosphorylation of mannose to mannose-6-phosphate. The second gene, *manA,* encodes a mannose-6-phosphate isomerase which converts the mannose-6-phosphate to the corresponding fructose-6-phosphate. The function of the third gene, *yjdF,* is as yet unknown. Upstream and in the same orientation of these three genes, a regulatory gene, *manR,* is located which encodes for the transcriptional regulator protein referred to ManR.

The mannose-operon is a positively regulated catabolic operon and is controlled by two different promoters. One promoter, *manR* promoter (*PmanR*), is responsible for the transcriptional regulator protein ManR. The second promoter, *manP* promoter (*PmanP*), is responsible for the transcription of the genes *manP-manA-yjdF* (jointly referred to "*manPA-yjdF*"). In the presence of mannose and in the absence of glucose ManR binds to *PmanP* and activates the expression of *manPA-yjdF.* Surprisingly it has been found, that ManR is not only the transcriptional regulator protein for the *manPA*-yjdF-promoter but is an auto-regulator for *manR* itself.

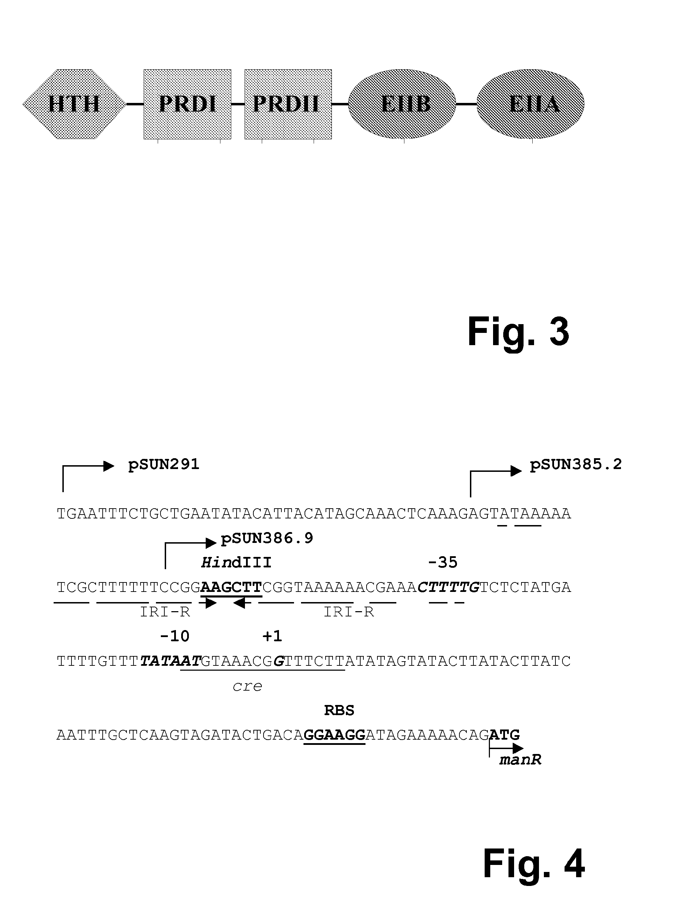

Figure 3 shows schematically the structure of the transcriptional regulator protein ManR and potential phosphorylation sites. As shown, ManR comprises two PRD (<u>P</u>TS <u>r</u>egulatory <u>d</u>omain) domains, one EIIA and EIIB domain as well as a HTH (Helix-turn-Helix) domain. PRDs are conserved regulatory domains present in transcriptional regulator proteins which can be phosphorylated in the course of carbon catabolism. The EIIA and EIIB domains are binding sites of the phosphoryl group transferred by ManP, the EII transporter of the mannose operon. HTH is a structural motive in a protein capable of binding DNA.

In the absence of the inducer mannose the EIIA and EIIB and eventuelly PRDI domain of ManR is phosphorylated by ManP, the mannose specific EII of mannose operon, and thereby rendered inactive.

Consequently, according to the first approach of the present invention deactivation of ManR in the absence of the inducer, mannose, is prevented, if the phosphorylation sites in EIIA and/or EIIB of ManR are manipulated or the domains deleted, so that it cannot accept a phosphoryl group.

To this, gene *manR,* in the genome of the bacterial host cell, is genetically altered by deleting or manipulating the corresponding nucleic acid sequence encoding the respective phosphorylation site(s) in ManR.

Further, according to the second approach of the present invention deactivation of the promoters of the mannose operon is prevented by interruption of the mannose transport, which can be effected by deletion or inactivation of the gene encoding ManP, in the genome of the bacterial host cell.

This alteration can be done by altering or deleting in the bacterial host cell the coding sequence of the genes encoding the proteins involved in the phosphorylation of the transcriptional regulator protein, here, in the mannose operon, *manR* and/or *manP.*

Such genetically altered knock-out host cells can be prepared according to any of the various method known in the art for such purpose. For example, homologous recombination vectors containing homologous targeted gene sequences 5'and 3' of the targeted nucleic acid deletion sequence can be transformed into the host cell. Upon homologous recombination the desired knock-out cell can be produced.

Gene knock-out methods are well known in the art. For example, gene inactivation by insertion of a polynucleotide has been described in, for example, Röder D. L. et al., "Marker-exchange mutagenesis of a pektate lyase isozyme gene in Erwinia chrys anthemy" J. Bacteriol. (1985) 164 (1: 51 - 56). Specific mutations or deletions in a gene can be constructed using cassette mutagenesis, for example, as described in Wells J. A. et al., "Cassette mutagenesis: an efficient method for generation of multiple mutations at defined sites", Gene (1985) 34 (2 - 3): 315 - 323; whereby direct or random mutations are made in a selected portion of a gene, and then incorporated into the chromosomal copy of the gene by homologous recombination.

It has been found by the present inventors that the promoters regulating the mannose operon, namely *PmanR* and *PmanPA-yjdF* are strong promoters which are, therefore, promising candidates for the production of heterologous polypeptides. However, mannose, the carbon source inducing these promoters, is a high price sugar which, as yet, restrict the use of mannose inducible promoters.

Since according to the present invention for expression of a heterologous polypeptide in a bacterial host cell no inducing carbon source is necessary for initiating expression also the use of promoters such as the mannose promoters are competitive not only in view of strongness but also in costs.

Consequently the present invention relates also to the use of the promoters of the mannose operon, *PmanR* and *PmanPA-yjdF,* as a promoter for controlling a heterologous nucleic acid sequence encoding for a target polypeptide in the recombinant bacterial host cell of the present invention.

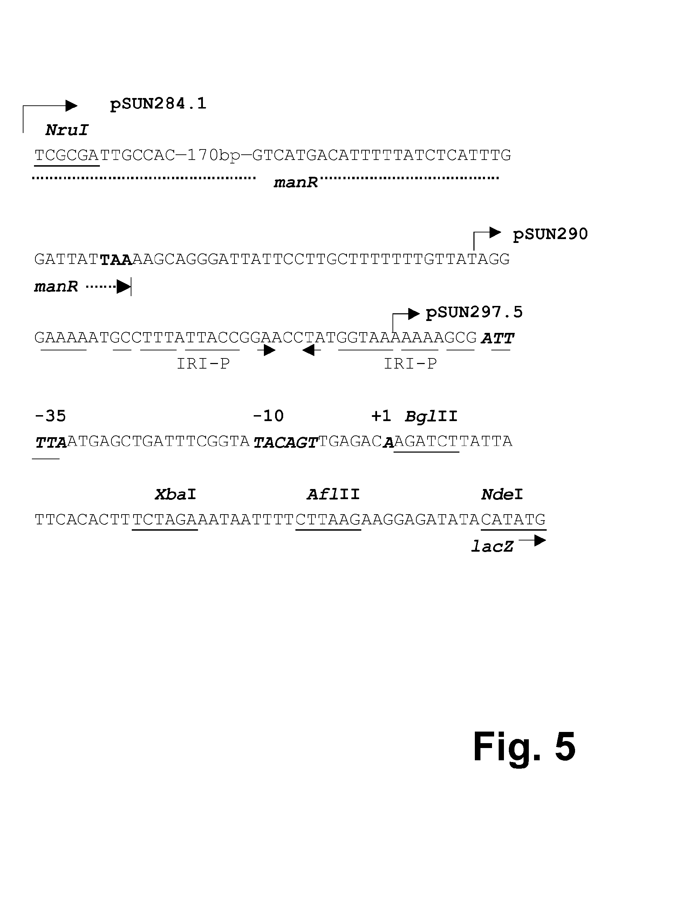

The nucleic acid sequence from *B.subtilis* comprising the promoter region of the *manP* promoter as used in various expression vectors like pSUN284.1 is shown in figure 5 with the transcription start site at an adenine nucleotide being highlighted, the -35 and - 10 boxes in italics and bold, and the end of *manR* marked by an arrow and the restriction sites *Bg*/II, XbaI, *Af*/II, *Nde*I and *Nru*I underlined. The start codon of the reporter gene *lacZ* is indicated.

The nucleic acid sequence obtained from *B.subtilis* comprising the promoter region of *manR* promoter is shown in figure 4 with the transcription start site being highlighted, the -10 and -35 boxes being italics and bold, the start of *manR* gene being indicated by an arrow and the *Hind*III restriction sites being in bold and underlined and the putative cre sequence being underlined.

With "promoter regions of the mannose operon" are meant the promoter regions which regulate expression of *manPA-yjdF* as well as of *manR* with or without the cre sequence.

The *"manPA-yjdF* promoter" as referred to herein consists essentially of the -35 region, the -10 region (Pribnow box), the transcription start site, and the ManR binding site.

The "*manR* promoter" as referred to herein consists essentially of the -35 regions, the - 10 region, the transcription start site and the ManR binding site and, optionally, a *cre* sequence.

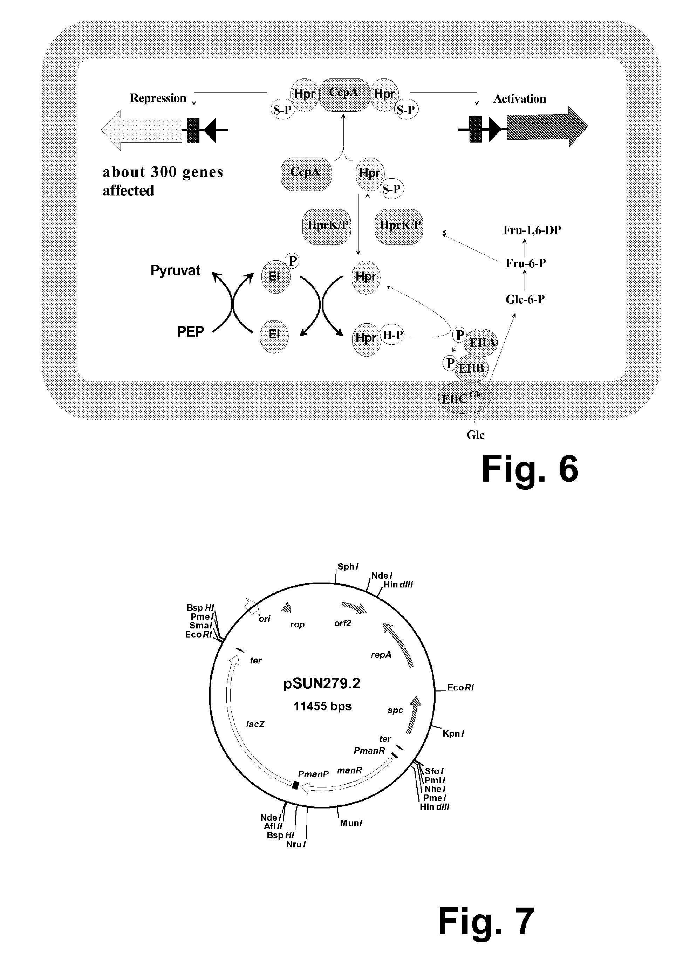

The cre sequence (catabolite repressive element) is a conserved 14 nucleotides long DNA sequence to which binds CcpA (catabolite control protein A), the global regulator protein of CCR in Firmicutes. The mechanism of catabolite repression and activation in *B.subtilis* is shown in figure 6. By the binding of CcpA, complexed by Serin-46 phosphorylated HPr (histidine protein) to the cre sequence the promoter is repressed. This represents a second mechanism of catabolite repression of the mannose operon. By this way, the expression of the regulator gene *manR* is repressed in the presence of glucose. In consequence, this inhibits an activation of the *manP* promoter. In the presence of glucose cells take up glucose via PtsG, a PTS-dependent transport system similar to the EII mannose transporter. In the cell the intermediates fructose-6-phosphate (Fru-6-P) and fructose-1,6-biphosphate (Fru-1,6-DP) accumulate and stimulate the HPr-kinase. HPr-Kinase (HPrk) phosphorylates HPr at Serin-46. Ser-46-HPr forms a complex with the DNA binding protein CcpA which binds to the socalled cre-sites present in many catabolite repressed and catabolite activated promoters of B. subtilis. Is the cre site downstream of the -10 promoter sequence or overlapping the - 10 sequence the binding of the Ser-46-HPr/CcpA complex inhibits expression from this promoter. This situation is found at the *manR* promoter (fig. 4).

The nucleic acid sequence comprising the promoter region of *manPA-yjdF* preferably comprises the nucleic acid sequence of figure 5 from bp-80 to the start codon of *lacZ* (SEQ ID NO.1) and more preferably the nucleic acid sequence of figure 5 from bp-80 and inclusive bp-1, i. e. upstream of the transcription initiation site A at bp+1 (SEQ ID NO. 2).

The nucleic acid sequence comprising the promoter region of *manR* preferably comprises the nucleic acid sequence of figure 4 from bp-122 to the start codon of *manR* (SEQ ID NO.3), more preferably, the nucleic acid sequence of figure 4 from bp-122 and bp+7, i. e. inclusive the putative cre-sequence, (SEQ ID NO. 4), and, in particular, the nucleic acid sequence of figure 4 from bp-122 and bp-1, i. e. upstream of the transcription initiation site G at bp+1 (SEQ ID NO. 5). Both, the promoter regions of *manP* and *man*R, comprise a binding site for the transcriptional regulator protein ManR (marked in figure 4 as IRI-R and in figure 5 as IRI-P), which is the transcriptional activator for the promoters of the mannose operon.

According to a further aspect, the present invention relates to such a genetically altered bacterial host cell comprising a vector with a nucleic acid sequence encoding for a polypeptide of interest operably linked to a carbon source inducible promoter, wherein the promoter of the vector is regulated by the transcriptional regulator protein for which the bacterial host cell has been genetically altered to be unable to deactivate in the absence of the carbon source specific for said transcriptional regulator protein.

Referring to the mannose operon above, the promoter of the vector can be the *PmanR* or *PmanPA-yjdF,* for example, any of the sequences SEQ ID NOs. 1 to 5 as well as any of the table above.

According to an embodiment of the present invention in the vector used in the present invention the genetically altered gene encoding for the respective transcriptional regulator protein incapable to bind a phosphoryl group transferred by the respective EII, can be incorporated. Consequently, in the recombinant bacterial host cell the transcriptional regulator protein is not only expressed by the chromosomal gene but also by the corresponding gene integrated into the vector which results in a higher concentration of transcriptional regulator protein and improved induction. For example, when the promoter of the vector is a promoter of the mannose operon, the gene *manR* can be integrated into the vector, wherein the nucleotide sequence coding EIIA domain has been deleted *(manRΔEIIA).*

A vector suitable for the present invention is preferably an autonomously or self-replicating plasmid, a cosmid, a phage, a virus or a retrovirus. A wide variety of host/vector-combinations may be employed for the present invention.

Useful expression vectors, for example, may consist of segments of chromosomal, non-chromosomal and/or synthectic nucleic acid sequences.

Suitable vectors include vectors with specific host range such as vectors specific for e. *g. B.subtilis* and *E.coli*, respectively, as well as vectors with broad-host-range such as vectors useful for gram-positive bacteria and gram-negative bacteria. "Low-copy", "medium-copy" as well as "high-copy" plasmids can be used.

For example, in *B.subtilis* a low-copy plasmid is pAMbeta1, medium copy plasmids are pBS72 derivatives and a high copy plasmid is pUB110. Useful vectors for, e. g. expression in *E.coli* are pBR322, pUC18, pACYC177, pACYC184, pRSF1010 and pBW22 or derivatives thereof such as plasmid pBLL 15 or plasmid pAKL15E.

The genetically altered bacterial host cell of the present invention is incapable of deactivating the transcriptional regulator protein controlling the promoter in the absence of the inducing carbon source of said promoter. Since the promoter of the vector is controlled by the same transcriptional regulator protein as the respective chromosomal promoter expression of the heterologous nucleic acid sequence encoding for the target polypeptide continues, even in absence of inducing carbon source.

Further, provided with the present invention is a method for producing a heterologous polypeptide in the genetically altered bacterial host cell of the present invention comprising the steps of

a) culturing under conditions allowing expression of the polypeptide, the genetically altered bacterial host cell of the present invention transformed with a vector comprising the nucleic acid sequence encoding the polypeptide operably linked to a promoter, wherein the promoter of the vector is regulated by the transcriptional regulator protein which can not be deactivated in the absence of the carbon source specific for the transcriptional regulator protein, and

b) recovering the polypeptide from the cells or from the cell culture.

According to the present invention expression of the target polypeptide starts automatically at the point of time when the medium runs out of the primary carbon source, or the concentration of primary carbon source decreases below a level required for CCR.

For allowing the cells to grow after induction, the primary carbon source can be fed to the medium further on at a level which does not allow carbon catabolite repression. For example, primary carbon source is fed in an amount which is immediately consumed by the cells so that in the fermentation broth essentially no primary carbon source is present which could stimulate CCR.

Generally it can be said that if the amount of primary carbon source present in the medium exceeds 1.0 g/l carbon catabolite repression is observed, whereas, if the amount is 0.01 g/l or less there is no carbon catabolite repression.

Since the borderline of carbon catabolite repression and induction of the promoter correlates with the growth rate of the cells, which in turn, correlates to the amount of primary carbon source fed to the medium, or present in the medium, the amount of primary carbon source, which does not cause carbon catabolite repression, can be adjusted by monitoring the growth rate. If at a given growth rate carbon catabolite repression occurs the amount of primary carbon source added to the medium must be reduced, thereby reducing the growth rate until a value at which no carbon catabolite repression is observed.

For most fermentation processes in the induction phase adjustment of the primary carbon source addition to an amount is suitable which results in a specific growth rate of $\mu \leq 0.2$ h$^{-1}$.

In any case, for a specific fermentation process a suitable value for the specific growth rate $\mu$ can be readily determined by routine work.

The vectors used, as well as construction and transformation of the host are as defined above.

As cell culture system continuous or discontinuous culture such as batch culture or fed-batch culture can be applied in culture tubes, shake flasks or bacterial fermentors etc. Preferably, in the induction phase when the target polypeptide is expressed, the primary carbon source is exponentially fed to the cultivation medium.

For culturing the genetically altered bacterial host cells conventional media as known in the art can be used such as complex media like "nutrient yeast broth medium", a glycerol containing medium as described by Kortz et al., J. Biotechnol. (1995) 39: 59 - 65, a mineral salt media is described by Kulla et. al. Arch. Microbiol. (1983) 135: 1, a LB-medium as described by Bertani et al., J. Bacteriol. 1951) 62: 293 - 300, or a batch medium for *E.coli* fermentation as described by Wilms et al., Biotechnol. Biong. (2001) 73:95-103.

The medium comprises a suitable carbon source, for example a sugar such as glucose for growing the host cell to a desired cell density. As carbon source a primary carbon source for the respective host cell is used which is different from the inducer, which is a secondary carbon source for said host cell.

The medium might be modified as appropriate, for example, by adding further ingredients such as buffers, salts, vitamins, amino acids, antibiotics or other micronutrients as are generally known to those of skill in the art. As well different media or combinations of media can be used during the culturing of the cells.

In one embodiment of the invention casamino acids are added to the culture medium. It has been found, that the presence of casamino acids in the medium can help to suppress basal level expression.

Typically, the casamino acids can be added in an amount of 0.05% to 0.1 % (w/v).

According to an embodiment of the present method for producing a heterologous polypeptide a bacterial host cell can be used comprising the genes encoding for the mannose operon in its genome, that is for which mannose is a secondary carbon source, and for which, for example, glucose is a primary carbon source. To be suitable as a host for the present method the bacterial cell is rendered incapable to deactivate ManR, the transcriptional regulator protein controlling the promoters of the mannose operon.

For example, the nucleotide sequence *manP* coding for ManP can be deleted, thereby inhibiting deactivation of ManR by phosphorylation via ManP.

Into the *manP* deleted bacterial host cell a vector is introduced comprising promoter P*manR* or promoter *PmanP* operably linked to the nucleic acid sequence encoding the target polypeptide.

The recombinant bacterial host cell is grown in a culture medium suitable for said bacterial host cell, wherein the culture medium can contain glucose and/or glucose can be fed to the culture medium in an amount sufficient to allow the bacterial host cell to multiplicate and to repress expression of the target polypeptide by glucose mediated carbon catabolite repression of the mannose promoter contained in the vector.

As soon as the glucose level of the medium drops below a value required for carbon catabolite repression, carbon catabolite repression of the mannose promoter of the vector is released and the mannose promoter starts expression of the polypeptide without the need of induction by mannose. Further, since in the bacterial genome the nucleic acid sequence encoding for ManP is deleted the mannose promoter cannot be deactivated by phosphorylation via ManP. Thus, the present method allows expression of a target polypeptide under control of a specific carbon source inducible promoter without the presence of the inducing carbon source.

Typically, during the induction phase in the fermentation process using a bacterial host cell transformed with a vector comprising a promoter of the mannose operon glucose, being the primary carbon source of the promoters of the mannose operon, can be fed to the fermentation medium in an amount resulting in a specific growth rate $\mu \leq 0.2$ h$^{-1}$. Further, exponential feed is preferred.

In principle, the explanations set out above with respect to the first alternative of the present invention requiring no inducing carbon source for inducing the promoter at all, are also applicable on the second alternative of the present invention according to which the amount of carbon source necessary for induction is reduced.

The present invention is advantageous in that the promoters controlling the expression of the heterologous nucleic acid sequence encoding for the target polypeptide allow a tight regulation.

Addition or presence of the inducing carbon source is not necessary.

According to the first alternative of the present invention the promoter is in the active state even without inducing carbon source, that is in the absence of inducer.

As shown in the following examples the present invention provides for a production of heterologous polypeptides by culturing the genetically altered bacterial host cell of the present invention wherein during the growth of the genetically altered bacterial host cells and prior to induction only very low or no premature expression of the polypeptide occurs, that is, there is essentially no leakiness of the promoter controlling the expression of the genes encoding for the polypeptide. Further, production of the polypeptide starts immediately upon auto-induction in high rates of productivity with a high end productivity.

Also shown is an example for the further alternative of the present invention wherein in the genome of the bacterial host cell harboring a vector with a mannose inducible promoter and a heterologous nucleic acid sequence, the gene is deleted, encoding for the mannose-6-phosphate isomerase.

The foregoing description will be more fully understood with reference to the following examples. Such examples are however exemplary of methods of practicising the present invention and are not intended to limit the scope of the invention.

**I) Isolation and identification of promoter regions of *manR* promoter and *manP* promoter of mannose operon**

If not stated otherwise the following materials and methods has been used:

**Bacterial strains and growth conditions**

*E. coli* JM109 (Yanisch-Perron C. et al., Gene 33, 1985, 103-119) and *Bacillus subtilis* 3NA (Michel J. F. et al., J. Appl. Bacteriol. 33, 1970, 220-227) were used as main hosts for cloning and expression. *E. coli* was grown in LB liquid medium (Luria S. E. et al., Virology 12, 1960, 348-390) and LB agar plates supplemented with 100 $\mu$g ml$^{-1}$ ampicillin or spectinomycin at 37 °C. *B. subtilis* was grown in LB liquid medium and C or S minimal medium at 37 °C (Martin-Verstraete I. et al., J. Mol.Biol. 214, 1990, 657-671). Liquid media and agar plates were supplemented with 100 $\mu$g ml$^{-1}$ spectinomycin, 10 $\mu$g ml$^{-1}$ kanamycin or 5 $\mu$g ml$^{-1}$ erythromycin, respectively.

For induction of the mannose promoter, sterile filtered or autoklaved D-mannose was added to a final concentration of 0.2 % w/v.

**Materials**

All chemicals were obtained from Sigma-Aldrich (Taufkichen, Germany), Fluka (Buchs, Germany) or Merck (Darmstadt, Germany). Synthetic DNA oligonucleotides were purchased from Eurofins MWG Operon (Ebersberg, Germany). Restriction enzymes and DNA modifying enzymes were purchased from Roche Applied Science (Mannheim, Germany) or New England Biolabs. (Frankfurt am Main, Germany). PCRs were run with High Fidelity-DNA polymerase from Fermentas (ST. Leon-Rot, Germany) on a MiniCycler from Biozym.

**Preparation of DNA and transformation**

DNA-isolation from *E.coli* and *B.subtilis* or from agarose gel were carried out with DNA preparation kits of Qiagen (Hilden, Gemrany) or Roche (Mannheim, Germany) as described by the manufacturer. Standard molecular techniques were used throughout the examples.

*E.coli* was transformed with plasmid DNA as described by Chung C.T. et al., Proc. Natl. Acad. Sci. USA 86, 1989, 2172-2175. *B.subtilis* was transformed with plasmid DNA according to the modified "Paris method" (Harwood C.R. Molecular Biological Methods for Bacillus, 1990, John Wiley & Sons Ltd., England).

**β-galactosidase activity measurement**

0.1 ml of the cells to be examined were treated with 900 μl Z-buffer and 10 μl toluene for 30 min at 37 ˚C. The β-galactosidase activity was determined with o-nitrophenyl-β-galactopyranoside at 22 ˚C according to Miller's method (Miller J. H., 1972, experiments in molecular genetics, Cold Spring Harbor, NY).

**Oligonucleotides (primers) used (Sequences ID NOs. 6 to 41)**

Table 1.

| Oligonucleotide | Sequence | Purpose |
|---|---|---|
| s4693 seq id no 6 | 5'-AAA AAA ACG CGT GTT TAA ACT GAA TTT CTG CTG AAT ATA CA-3' | PCR amplification of *manR* from *B. subtilis* |
| s4694 seq id no 7 | 5'-AAA AAA TCT AGA AAG TGT GAA TAA TAA GAT CTT G-3' | PCR amplification of *manR* from *B. subtilis* |
| s4802 seq id no 8 | 5'-AAA AAA ACT AGT GTT TAA ACA GGG AAA AAT GCC TTT ATT AC-3' | Forward primer for amplification of P*manP*□□ |
| s4833 seq id no 9 | 5'-AAA AAA GTT TAA ACC CCT GGC GAA TGG CGA T-3' | Amplification of spc from plasmid pDG1730 |
| s4835 seq id no 10 | 5'-AAA AAA GAA TTC ATT AGA ATG AAT ATT TCC CAA AT-3' | Amplification of spc from plasmid pDG1730 |
| s4956 seq id no 11 | 5'-AAT TGC GTC GAG ACC CCT GTG GGT CTC GTT TTT TGG ATC CGG CGC CCA CGT GGC TAG CC-3' | Insertion of *tufA* terminator |
| s4957 seq id no 12 | 5'-TTA AGG CTA GCC ACG TGG GCG CCG GAT CCA AAA AAC GAG ACC CAC AGG GGT CTC GAC GC-3' | Insertion of *tufA* terminator |
| s5006 seq id no 13 | 5'-**Cy5**-TAG CCT TTT TTA TAG TTG TTC AGC CAC TGT-3' | Labeled primer for primer extension |

(continued)

| Oligonucleotide | Sequence | Purpose |
|---|---|---|
| s5007 seq id no 14 | 5'-**Cy5**-ATC CAC GCC ATA ATG CAT GCC GCC ATT AAT-3' | Labeled primer for primer extension |
| s5019 seq id no 15 | 5´-tta agC TCT TAA GGAG GAT TTT AGA ATG GCT AAA GAA AAA TTCg-3´ | tufA TI-Region |
| s5020 seq id no 16 | 5´-tta agG AAT TTT TCT TTA GCC ATT CTA AAA TCC TCC TTA AGA Gg-3´ | tufA TI-Region (compl.) |
| s5069 seq id no 17 | 5'-AAA AAA GAA TTC GAT ATC AGA TCT ACG CGT TAA CCC GGG C-3´ | PCR erythromycin resistance gene |
| s5070 seq id no 18 | 5'-AAA AAA CAA TTG AAT CGA TTC ACA AAA AAT AGG-3´ | PCR erythromycin resistance gene |
| s5071 seq id no 19 | 5'-AAA AAA AGA TCT CAT GGC AGG GCT TGA GAA-3' | *manA* deletion |
| s5072 seq id no 20 | 5'-AAA AAA GAA TTC TTA TTT ACC TCT GTG CTT CTT-3´ | *manA* deletion |
| s5097 seq id no 21 | 5'-Cy5-CACTGTACCCTATCTGCGAAA-3' | Labeled primer for primer extension |
| s5098 seq id no 22 | 5'-Cy5-ATTGAGATAATCCTCGATCACTT-3' | Labeled primer for primer extension |
| s5139 seq id no 23 | 5'-aaa aaa tga tca TTA CTT GTA CAG CTC GTC-3' | f-Primer *PmanP-eGFP* |
| s5156 seq id no 24 | 5'-aaa aaa tga tca ccg gtC GAT TGC CAC ATT AAA GG-3' | r-Primer *PmanP-eGFP* |
| s5203 seq id no 25 | 5'-GATATCCTGCACCATCGTC-3' | Backward primer for amplification of $P_{manP}$ for promoter study |
| s5208 seq id no 26 | 5'-GGTACCATTTCTTGCTGAATA-3' | Amplification of $P_{manR}$-region from pSUN279.2 |
| s5209 seq id no 27 | 5'-CTTAAGCCTGTCAGTATCTACTTGAG-3' | Amplification of $P_{manR}$-region from pSUN279.2 |
| s5234 seq id no 28 | 5'-aaa aaa ccg CTC GTC TTC CTA AGC ATC CT-3' | f-Primer rep (pUB110) |
| s5235 seq id no 29 | 5'-aaa aaa gaa tTC GAG ATC AGG GAA TGA GTT T-3' | r-Primer rep (pUB110) |
| s5236 seq id no 30 | 5´-tta agA ATT AAA GGA GGA ATT CAA AAT GGC AGA CAA TAA CAA Ag-3´ | gsiB TI-Region |

(continued)

| Oligonucleotide | Sequence | Purpose |
|---|---|---|
| s5237 seq id no 31 | 5′-gat ccT TTG TTA TTG TCT GCC ATT TTG AAT TCC TCC TTT AAT Tc-3′ | gsiB TI-Region (compl.) |
| s5262 seq id no 32 | 5'-AAA˙AAA GCT AGC GTT TAA ACA AAA AGC GATT TTA ATG AGC TG-3' | Forward primer for amplification of $P_{manP\square}$ |
| s5362 seq id no 33 | 5′-GGT ACC CCC GGG TAG CCT GGA TGG ATC AGA A-3′ | manP deletion |
| s5363 seq id no 34 | 5'-ACT AGT GAA TTC CTT TTC CAA TCG CA-3' | manP deletion |
| s5407 seq id no 35 | 5'-AAA AAA GGC GCC GCT AGC TGG AGA ATA TAA CGG TT-3′ | manP deletion |
| s5408 seq id no 36 | 5'-ACA CTC CTT AAG TCT AGA AA-3' | manP deletion |
| s5617 seq id no 37 | 5'-GGA GGG GAG AAA ACA CCT A-3' | manA deletion |
| s5618 seq id no 38 | 5'-AAA AAA GAT ATC TCA AGA AAA TCC CCC GCT TT-3' | manA deletion |
| s5932 seq id no 39 | 5′-AAA AAA GCT AGC GTT TAA ACA GTA TAA AAA TCG CTT TTT TCC-3′ | Forward primer for amplification of $P_{manR\square}$ |
| s5933 seq id no 40 | 5′-AAA AAA GCT AGC GTT TAA ACC GGA AGC TTC GGT AAA AA-3′ | Forward primer for amplification of $P_{manR\square}$ |
| s5934 seq id no 41 | 5'-GTG CAG GAG CTC GTT ATC-3' | Reverse primer for amplification of $P_{manR\square}$ |

**Experiment 1:**

Isolation of DNA fragment carrying the promoter regions of the mannose operon and determination of transcription initiation sites of *manR* promoter and *manP* promoter.

Chromosomal DNA of *Bacillus subtilis* 168 was isolated by using DNeasy Blood & Tissue Kit of Qiagen (Hilden, Germany).

A DNA fragment of about 2.3 kb with the complete *manR* gene and the *manR* promoter and the intergenic region between *manR* and *manP* with the *manP* promoter was amplified from the obtained DNA by PCR using primer s4693/s4694.

The obtained DNA fragment of about 2.3 kb was used for a primer extension experiment for determining the transcription initiation sites of *manR* promoter and *manP* promoter.

For isolation of mRNA for primer extension a shuttle factor was constructed from the *E.coli* vector pIC20HE (Altenbuchner et al., 1992, Methods Enzymol. 216, 457-466) and the *B.subtilis* vector pUB110 (MacKenzie et al., 1986, Plasmid 15, 93-103). The vector contained the *lys* gene as reporter gene, which codes for the mature form of lysostaphin from *Staphylococcus simulans* (Recsai et al., 1987, Proc. Natl. Acad. Sci. USA 84, 1127-1131). Into this high copy pUB110 derivative the 2.3 kb DNA fragment was cloned upstream to the lysostaphin gene. The resulting plasmid was named pSUN178.4 and introduced into *Bacillus subtilis* 3NA.

*Bacillus subtilis* 3NA with plasmid pSUN178.4 was grown in LB medium with kanamycin. In the exponential growth phase the culture was induced with 0.2 % w/v mannose. After 1 hour growth at 37 °C the induced and non-induced cells were harvested. Total RNA was isolated with the Qiagen-RNeasy Mini Kit.

With Cy5 at the 5'-end labeled primers s5006, s5007, s5097 and s5098 were used. Primer s5006 and s5007 hybridized respectively from +21 to +50 and from +76 to +105 with respect to the start codon of lysostaphin gene. Primer s5097 and s5098 hybridized respectively from +81 to +101 and from +131 to +153 with respect to the start codon of *manR*.

The same primers were used for the sequencing reaction of plasmid DNA of pSUN178.4, which served as size standard. The AMV-Reverse Transcriptase and T7-DNA polymerase from Roche were used, respectively, for the reverse transcription and DNA sequencing. The products of reverse transcription and sequencing were analyzed on a denaturating polyacrylamide sequencing gel (GE healthcare). All other reagents used were provided by Amersham Pharmacia Biotech AutoRead Sequencing kit.

The transcription initiation site of manP-promotor was determined by using primer s5006. DNA sequence reactions of the plasmid pSUN 178.4 with the same primer were prepared and run on the same denaturing gel for comparison.

Figure 5 shows the DNA sequence around the *manP* promoter with the transcription initiation site at A (adenine nucleotide) being highlighted. The deduced -10 and -35 boxes are in italics, the end of the *manR* gene is marked by arrows, restriction sites for *Bg*/II *Nru*I, *Xba*I, *Nde*I *Afl*II are underlined. IRI-P indicates an imperfect inverted repeated sequence, the putative ManR binding site.

The transcription initiation site of *manR* promoter was determined with RNA isolation and DNA sequencing being carried out as described above with respect to *manP* promoter except that primer s5098 was used which binds in the *manR* gene.

In figure 4 the DNA sequence of the *manR* promoter region is shown with the transcription initiation site at G (guanine nucleotide) being highlighted, the deduced -10 and -35 boxes in italics, ribosomal binding site (RBS) underlined, and the start of the *manR* gene, respectively, being indicated by an arrow. The restriction sites and a putative cre sequence are underlined. IRI-R indicates an imperfect inverted repeated sequence, the putative ManR binding site.

The transcription from the *manR* promoter and in particular from the *manP* promoter was strongly increased when the cells were induced by mannose as was seen by the much stronger signals in the primer extension experiment.

The primers used are shown in table 1 above.

**Experiment 2**

The primer extension experiment according to Experiment 1 located the transcription initiation site of the *manP* promoter near the 3'-end of the intergenic region between *manR* and the beginning of *manP.* For determining the *manP* promoter region more precisely the 2.3 kb DNA fragment was shortened step-by-step by PCR-amplification, the obtained sequence fragments of different lengths cloned back to the same basic expression vector and expression was studied.

**a) Construction of basic expression vector**

An expression vector with promoterless *lacZ* as reporter gene was constructed. The expression vector was designed as a shuttle vector capable of replicating both in *B.subtilis* and in *E.coli* and named pSUN272.1.

The reporter gene *lacZ* was cut with *Nde*I and *Xma*I from pLA2 (Haldimann A. et al, 2001, J. Bacteriol. 183, 6384-6393) and ligated into pJOE5531.1, a derivate of the rhamnose inducible expression vector pWA21 (Wegerer et al., 2008, BMC. Biotechnol. 8, 2) which contained the *B.subtilis* tufA transcription terminator at the *Xma*I site. Into this plasmid a pair of oligonucleotides s4956/4957 was inserted between the *Afl*II/*Mun*I restriction sites in order to add the same *tufA* transcription terminator upstream of *lacZ.* So the "reading through" from plasmid promoters into *lacZ* as well as "reading through" out of *lac*Z into the flanking plasmid sequences was avoided by the terminators. A spectinomycin resistance gene spc for both *E.coli* and *B.subtilis* was amplified from plasmid pDG1730 (Geurout-Fleury et al., 1996, Gene 180, 57-61) with oligonucleotides s4833/4835 and inserted into the plasmid obtained above. In addition, the *E.coli* vector part was shortened by deleting a *Bsp*HI/*Hind*III fragment. Subsequently, an *Eco*RI/*Sph*I fragment with the replication region of *B.subtilis* pMTLBS72 (Lagodich et al., 2005, Mol. Biol. (Mosk) 39, 345-348) was ligated into the plasmid.

The 2.3 kb DNA fragment obtained in Experiment 1 was inserted into pSUN272.1 in front of *lacZ* by digesting with *Af*/II and *Nhe*I and ligation, thereby obtaining expression vector pSUN279.2 with the plasmid map as shown in figure 7. The primers used are shown in table 1 above.

**b) Determination of expression efficiency of vector pSUN279.2**

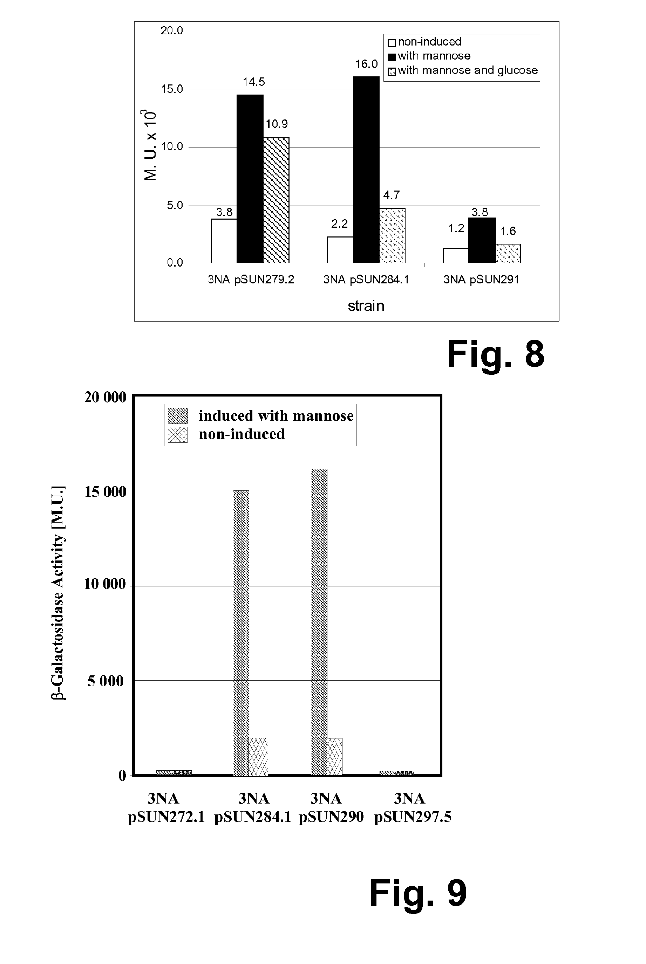

The plasmids pSUN279.2 and pSUN272.1 obtained in a) above were brought into *B.subtilis* 3NA. The latter served as background control. The *B.subtilis* 3NA strains carrying one or the other plasmid were grown in LB medium with spectinomycin and in the exponential growth phase either 0.2 % mannose, 0.2 % mannose plus 0.2 % glucose or no sugar (uninduced control) were added to the cultures for induction. After one hour induction the β-galactosidase activity of the cells was determined through Miller's assay. The results are shown in figure 8

The non-induced culture of *B.subtilis* containing pSUN279.2 showed already a quite high basal level of β-galactosidase activity. The presence of mannose resulted in a further 4-fold increase of β-galactosidase activity whereas the activity with mannose and glucose was reduced but was still quite above the basal level. The results clearly indicate that the promoter activity seen in pSUN279.2 could originate from the region between *manR* and *manP,* from the region upstream of *manR* or from both.

Therefore, the upstream region of *manR* as well as most part of *manR* were both deleted from pSUN279.2 by cutting the 2.3 kb DNA fragment of pSUN279.2 as shown in figure 7 between SfoI and *Nru*I to give plasmid pSUN284.1.

*B.subtilis* 3NA was transformed with this plasmid pSUN284.1 and the expression efficiency determined as set out above. The result is shown in figure 8. As can be seen from figure 8 this *manR* deleted vector pSUN284.1 in *B.subtilis* 3NA showed only about half of the basal level of β-galactosidase activity compared to pSUN279.2 in *B.subtilis* 3NA, an even stronger increase by mannose induction and again a stronger reduction in the presence of glucose. These results prove that the *manP* promoter is located between *manR* and *manP* and show that the chromosomal copy of *manR* is sufficient for regulating all *manP* promoter copies on the low copy plasmids.

#### c) Localization of *manP* promoter region

For localizing the promoter region of *manP* in addition to the shortened DNA fragment of pSUN284.1 further shortened sequence fragments were prepared from the 2.3 kb DNA fragment by amplifying DNA fragments by PCR shortened at different positions upstream to the transcription initiation site of *manP* promoter and inserting the fragments in pSUN272.1 as shown in figure 5.

Deletion down to bp -81 and bp -80 upstream to the transcription initiation site of *manP* resulted in a second deletion sequence comprising SEQ ID NO. 1.

A further deletion was carried out down to bp -41 and bp -40 upstream to the transcription initiation site of *manP* (third deletion sequence).

Plasmids comprising the second deletion sequence, pSUN290, and the third deletion sequence, pSUN297.5 were constructed in a similar way as plasmid pSUN284.1 in 2b) above, by inserting the PCR products amplified with primers s4802/s5203 and s5262/s5203, respectively, into pSUN272.1 via restriction enzymes *Eco*RV and *Nhe*I.

The plasmids were inserted into *B.subtilis* 3NA and cultured as set out above in b) After 1 hour induction the β-galactosidase activity of the cells was determined as set out in b) above. The results are shown in figure 9.

As shown in figure 9 none of the strains with pSUN290 and pSUN284.1 showed a significant difference concerning induction of *lacZ* by mannose. However, in *B.subtilis* 3NA comprising pSUN297.5 , induction by mannose was completely abolished and the basal expression level was nearly 0. From these results follows that the ManR binding site of the *manP* mannose promoter region is located between bp -80 and -35 with respect to the transcription initiation site of *manP.*

#### Experiment 3: Determination of *manR* promoter

#### a) Identification of cre sequence

Since most CCR in Firmicutes is mediated through catabolite control protein A (CcpA) a search for the respective binding sites (cre sequence) was carried out in the whole mannose operon using the DNA alignment function in the Clone Manager program. For the alignment the cre consensus sequence 5'-WWTGNAARCGNWWWCAWW-3' (Seq ID NO 42) was used.
Only in the promoter region of *manR* one putative cre sequence was found as shown in figure 4 which is located downstream to the -10 box.

#### b) Evaluation of expression efficiency of *manR* promoter

For evaluating the expression efficiency of the *manR* promoter an expression vector like pSUN284.1 was constructed as set out above and named pSUN291. To this, a DNA fragment including the putative *manR*-promoter and about 600 bp upstream of *manR* was amplified with primer s5208/s5209 and linearized plasmid DNA pSUN279.2 as template and inserted in front of *lacZ* in plasmid pSUN272.1, by digesting with *Kpn*I and *Afl*III and ligation.

The DNA-sequence is shown in figure 4.

Plasmid pSUN291 was introduced into B.subtilis 3NA and the β-galactosidase activity was measured as set out above in experiments 2 b).

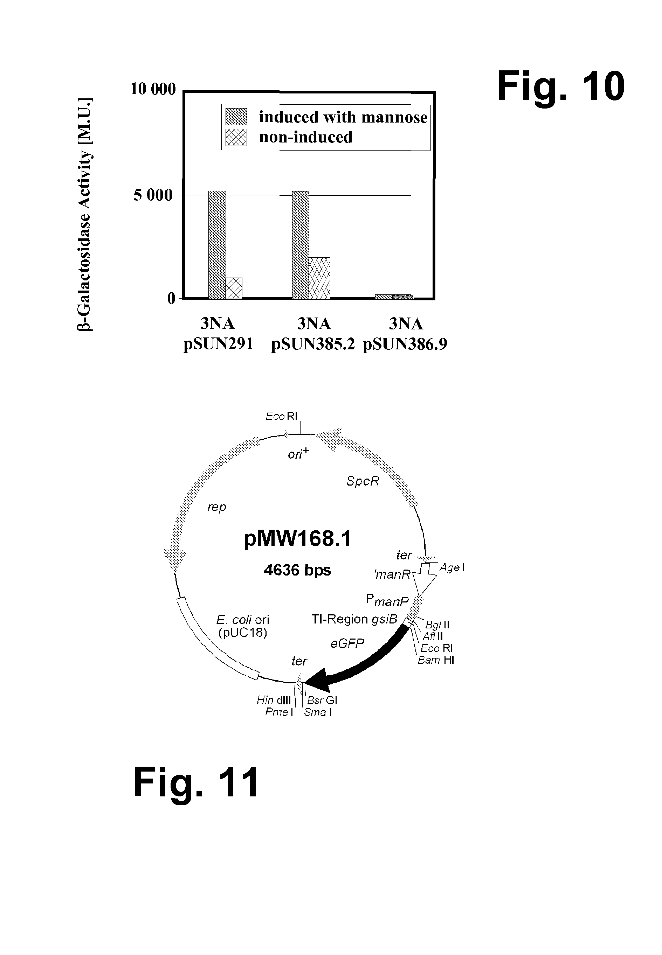

The result is shown in figure 10. Here, the basal expression was already relatively high and was further increased by the 3-fold by addition of 0.2 % mannose. Addition of glucose led to repression of β-galactosidase activity to nearly the basal expression level.

The result indicated that the *manR* promoter is not just a weak constitutive promoter but subject to mannose and CCR regulation.

### c) Localization of *manR* promoter region

As in experiment 2c) for further localization of the promoter region of *manR* DNA-fragments of different lengths were prepared from the DNA-sequence as contained in pSUN291 by PCR amplifying DNA with primers binding at different positions upstream to the transcription initiation site of *manR* promoter (primer s5932 and s5933) and a primer binding downstream in the *lac*Z gene (s5934) (figure 4).

A first deletion sequence was obtained by shortening the sequence shown in figure 4 down to bp -82 and bp -81 upstream of the transcription inition site G and the second deletion was obtained by shortening down to bp -62 and bp -61 upstream of the transcription initiation site G.

Analogous to experiment 2c) the PCR fragments were digested with endoR *Sac*I and *Nhe*I and ligated to pSUN279.2 DNA digested by the same restriction enzymes and the resulting plasmids named pSUN385.2 and pSUN386.9, respectively.

Each plasmid was inserted into *B.subtilis* 3NA and cultured as set out in experiment 2b. After one hour induction the β-galactosidase activity of the cells was determined as set out in experiment 2b. The results are shown in figure 10. There is no significant difference concerning induction of *lac*Z by mannose of *B.subtilis* 3NA comprising pSUN385.2 compared to pSUN291. However, in *B. subtilis* pSUN386.9 with the second deletion sequence, induction by mannose was completely abolished and the basal expression level was nearly 0. From this results follows that the ManR binding site of the *manR* promoter region is located between bp-81 and bp-35 with respect to the transcription initiation site of *manR.* The ManR binding site might even overlap the -35 sequence as it is found for classI activators since the inverted repeated sequence found in the proposed binding site extends into the proposed -35 sequence.

### II) Construction of recombinant host cell with genetically altered gene regions of mannose operon

### Experiment 4: Transformation

### a) Construction of plasmid pMW168.1 as expression vector

Using the nucleic acid sequence of the manP promoter region as introduced in plasmid pSUN284.1 as shown in figure 5 and as used in experiment 2c, plasmid pMW168.1 was constructed as set out below and introduced into *B.subtilis* 3NA as host.

A shuttle vector replicable in both *E.coli* and *B.subtilis* was designed as set out in Experiment 2a) with the exception that *eGFP* was used as reporter gene instead of *lac*Z. Also the transcription initiation region of *manP* was replaced by that of the gene *gsiB* (Stress protein; Jürgen et al., supra). Thereby also the start codon of *eGFP* and 6 codons following the start codon were replaced.

The schematical structure of the obtained promoter and transcription initiation region was as follows:

Shown is the arrangement of the genes (arrows) and the regions (boxes) with the relevant restriction sites.

The sequence (Seq ID NO 43) of the transcription initiation region of *gsiB* as used was:

5´-<u>cttaag</u>AATTAAAGGAGGAATTCAAA**ATG**GCAGACAATAACAAA<u>ggatcc</u> -3´

Af/II                    SD          Startcodon                    *Bam*HI

Generally, plasmid pMW168.1 was obtained as shown in the following flow chart:

In the flow chart the names of the vector-DNAs, the insert-DNAs and the complementary oligonucleotides used were as indicated in the boxes, with respect to the products of PCR the primers and the template-DNA were as within the brackets, the restriction enzymes used were indicated at the respective sites.

The cloning steps were carried out with *E.coli* JM109. The plasmids used were pUC18, a cloning vector for PCR products with amp-resistance (Yanosch-Perron et al., supra); pWA21 an expression and cloning vector for *E.coli* with amp-resistance (Wegerer et al., 2008, BMC Biotechnol. 8,2); pSUN202.4 a pUB110 derivate with *manP* promoter region and *amp* and *kan* resistance, being a shuttle vector for *E.coli* and *B.subtilis;* and pSUN266.1, a pUC18 derivate with integration site between *ter*-sequences and *spc* and *amp* resistance.

The plasmid pSUN266.1 is a derivative of rhamnose-inducible expression vector pWA21 where the rhamnose promoter and eGFP gene was replaced by a sequence containing two transcription terminators of the *tufA* gene of *Bacillus subtilis* in direct orientation and separated by restriction sites for *Bam*HI, *Sma*I and *Afl*II (see sequence below: Seq ID NOS 44 and 45) as well as a spectinomycin resistance gene. The later was amplified from plasmid pDG1730 (Cuerout-Fleury et al 1996) with the primers s4833 and s4835 (table 1). In the final construct pMW168 the mannose promoter and eGFP gene is inserted between the two *tufA* transcription terminator sequences.

*Bam*HI *Sma*I *Afl*II
AATTGCGT***CGAGACCCCTGTGGGTCTCGTTTTTTT***<u>GGATCCCCGGGACGT</u>***CGAGACCCCTGTG***
TTAACGCA***GCTCTGGGGACACCCAGAGCAAAAAA***CCTAGGGGCCCTGCA***GCTCTGGGGACAC***

*Pme*I     *Hind*III
***GGTCTCGTTTTTTT***<u>GTTTAAAC</u><u>AAGCTT</u>
***CCAGAGCAAAAAA***CAAATTTGTTCGAA

Nucleotide sequence of the two tufA terminator sequences (bold, italics) and restriction sites between and at the end of the sequence (underlined).

Replacement of the transcription initiation region inclusive the start codon and the codons following the start codon were carried out using complementary oligonucleotides and via the single restriction sites *Bgl*II, *Afl*II and *Bam*HI. The construction of the vector started with the replacement of the transcription initiation region of the T7 *gene 10* of vector pWA21 (Wegerer et al., supra) by the transcription initiation region of *tufA* from *B.subtilis* via complementary oligonucleotides s5019 and s5020, respectively. In further cloning steps this transcription initiation region was replaced by that of *gsiB* (Oligonucleotides s5236/s5237). The final plasmid pMW168.1 contained the *rep* gene inclusive ori+ from pUB110.

The plasmid map of pMW168.1 is shown in figure 11.

#### b) Insertion vectors

#### b1) Insertion vector for deletion of *manP*

Insertion vector pSUN356.7 was used to delete gene *manP* encoding for the mannose-specific EII from the mannose-operon on the chromosome of sporulation deficient *B.subtilis* NA and the obtained strain was named *B.subtilis* TQ356 (spo0A3 *manP::ermC*). As selection marker erythromycin resistance cassette was used. Vector pSUN356.7 is a pUC18 derivate (ampicillin resistance) and contains an erythromycin resistance gene flanked by sequences of *manR* and *manA* and outside the replacement cassette a spectinomycin resistance gene. The spectinomycin resistance gene was amplified from pDG1730 (see above for pSUN266.1). The erythromycin resistance gene was amplified from plasmid pDG1730 by the primer s5069 and s5070. The C-terminal end of the *manR* gene was amplified from *Bacillus subtilis* DNA by the primer s5407 and s5408 and inserted on one side of the erythromycin gene. The N-terminal end of *manA* was amplified from the *Bacillus subtilis* chromosome by the primer s5362 and s5363 and inserted at the other side of the erythromycin resistance gene.

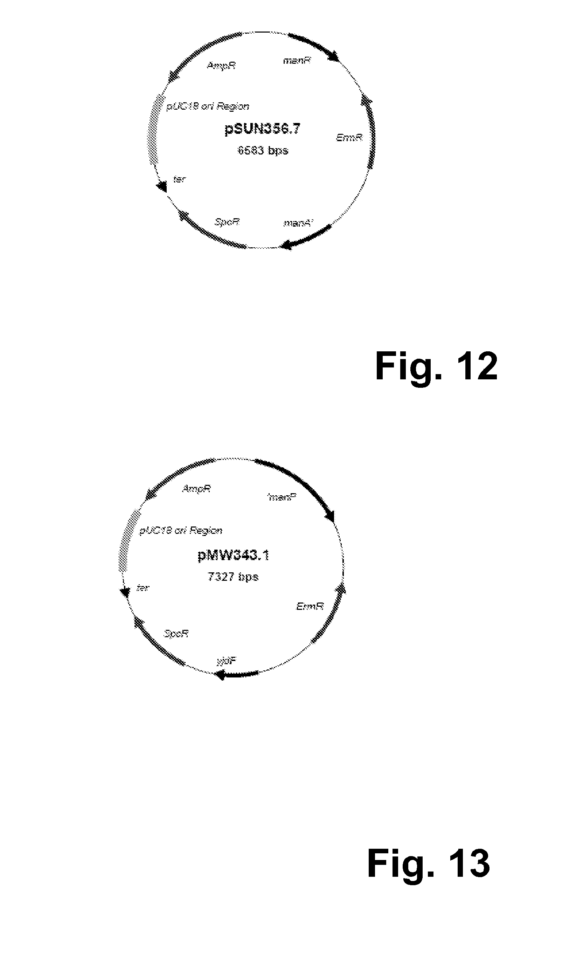

The map of plasmid pSUN356.7 is shown in figure 12.

#### b2) Insertion vector for deletion of *manA*

Further, a *B.subtilis* knockout mutant was produced with deleted *manA* from the mannose operon on the chromosome of sporulation deficient *B.subtilis* 3NA, thereby with insertion vector pMW343.1 obtaining *B.subtilis* MW343 (*spo0A3, manA::ermC*). Vector pMW343.1 is similar to pSUN356.7. This pUC18 derivative with an ampicillin resistance gene contains an erythromycin resistance gene flanked by sequences of *manP* and *yjdF* and outside a spectinoymcin resistance gene. The *manP* C-terminal sequence was amplified from *Bacillus subtilis* DNA by the primer s5617 and s5618 and inserted on one side of the erythromycin resistance gene. The *yjdF* N-terminal sequence was amplified from *Bacillus subtilis* DNA by the primer s5071 and s5072) and inserted on the other side of the erythromycin resistance gene.

Gene *manA* encodes for mannose-6-phosphate isomerase which converts mannose-6-phosphate to fructose-6-phosphate.

For monitoring successful deletion an erythromycin resistance cassette was used.

The map of plasmid pMW343.1 is shown in figure 13.

#### b3) Transformation

*B.subtilis TQ356* and *B.subtilis* MW343 obtained above in b1) and b2) where each transformed with plasmid pMW168.1 obtained above in a1).

**Media used:**

a) Minimal Glucose (MG):

| | |
|---|---|
| 2.0 g | $(NH_4)SO_4$ |
| 6.0 g | $KH_2PO_4$ |
| 14.0 g | $K_2HPO_4$ |
| 1.0 g | $Na_3Citrat$ |
| 0.2 g | $MgSO_4*7H_2O$ |
| 5.0 g | Glucose (separately as 20 - 50 % stock solution) |

b) Medium I:

| | |
|---|---|
| 9.50 ml | MG |
| 0.20 ml | Casaminoacids (1 % stock solution) |

(continued)

0.05 ml    MgSO$_4$ (1 M stock solution)

c) Medium II:

8.00 ml    MG
0.10 ml    Casaminoacids (1 % stock solution)
0.05 ml    MgSO$_4$ (1 M stock solution)

Transformation was carried out following the protocol of Anagnostopulos et al, "Requirements for transformation in Bacillus subtilis" J. Bacteriol. (1961) 81: 741-746. A single colony of each bacterial strain was given to 5 ml medium I and incubated at 37 ˚C in a roller overnight. 1 ml of the overnight culture (OD$_{600}$ between 1 and 2) was transferred into 8 ml medium II in a 100 ml baffled Erlenmeyer flask and incubated at 37 ˚C for 85 min. Then, 1 ml of competent cells were transferred into testtubes of Schütt Company and mixed with the respective insertion vectors. Prior to admixing with the competent cells the insertion vectors had been cut at a non essential site with a single cutter. The obtained mixture was precipitated by isopropanol and allowed to ligate at room temperature for at least 2 hours.
Then, the obtained transformed cells were incubated at 37 ˚C for 30 min in a roller, centrifugated at 4,500 rpm for 5 min at room temperature. The pellet was resuspended in residual liquid and plated.

**Experiment 5: Fermentation**

**5. 1 Materials and methods**

In general, also for the fermentation experiments standard molecular techniques were used if not stated otherwise.

Optical density

For determining the optical density (OD) the spectrophotometer Ultrospec 1100pro of Amersham Bioscience Company was used at 600 nm in accordance to the protocol of the manufacturer.

Determination of the dry biomass concentration

For the determination of the dry biomass concentration cx moisture meter MB 835 Halogen of Ohaus Company was used.

Spectophotometrical measurement of fluorescence

Expression and fluorescence, respectively, of eGFP was analyzed by the Multifunction reader GENios of TECAN Company using the reader software X Fluor 4 (version V4.11) with the following measuring parameters:

| Measuring parameter | value |
| --- | --- |
| Excitation filter | 485 nm |
| Emission filter | 535 nm |
| Gain (manual) | 60 |
| Integration time | 50 msec |
| Number of flashes | 3 |
| Read mode | Top |

Online fluorescence measurement in fermentor

During the fermentation the expression of eGFP was monitored online using the fluorescence probe (Micropack HPX-2000, High Power Xenon Lightsource of Ocean Optics, Inc.; S2000 fiber optic spectrometer).

The measuring parameters were as follows: excitation filter 485 nm, emission filter 535 nm, filter 0.6). For recording and storage Ocean Optics SpectraSuite Software was used.

Fluorescence is indicated as relative fluorescence unit (RFU). Shortly before obtaining 4,000 RFU the integration time of 50 ms was changed to 25 ms and then to 10 ms. In these cases the measuring values were multiplicated by factor 2 and 5, respectively.

Cultivation of pre-cultures

A single colony on a LB agar plate was used to inoculate an overnight culture in 5 ml Spizizens minimal medium (SMM) including 0.05 % (w/v) Casamino acids (CA) and antibiotic. 1 ml of the overnight culture was added to 20 ml SMM with 0.05 % (w/v) CA and antibiotic and incubated 5-6 h at 37 °C in a 250 ml Erlenmeyer flask (pre-culture 1). 10 ml of pre-culture 1 were added to 200 ml preculture medium 2 including 5 g/l glucose and incubated up to 8 h at 37 °C in a 1 l Erlenmeyer flask (pre-culture 2). For inoculation of the fermentors pre-culture 2 with an $OD_{600}$ between 1 and 2 was used.

Fermentation

In general, fermentation was carried out in accordance to the principles of Wilms et al., 2001, Biotechnol. Bioeng. 73, 95-103.

As soon as glucose, the carbon source, was completely consumed the batch mode was switched to the fed-batch mode.

In the fed-batch phase the feed solutions I and II were fed exponentially in an amount to adjust a constant growth rate of $\mu=0.10$ h$^{-1}$. The feed media I and II were added in proportion to their overall volumes, i.e. 4.2:1.0 (corresponding to 80.8 % medium I and 19.2 % medium II of the overall feed F). In the fed-batch phase catabolite repression by glucose is avoided due to the immediate consumption of glucose by the cells.

The total feed F at time t was calculated by the following formula:

$$F(t) = \left[ \frac{\mu_{set}}{Y_{X/S}} + m \right] \cdot \frac{C_{x0} \cdot V_0}{C_{s0}} \cdot e^{\mu_{set} \cdot t}$$

with

m = maintainance coefficient· (0.04 g g$^{-1}$ h$^{-1}$)
$Y_{x/s}$ = specific yield coefficient of biomass related to substrate (0.5 for glucose)
$C_{x0}$ = biomass concentration at start of fed-batch phase
$V_0$ = reactor volume at start of fed-batch phase (=batch volume)
$C_{s0}$ = glucose concentration in feed solution

Table 2: Media used

| medium | component | concentration |
|---|---|---|
| | | |
| Spizizens Minimalmedium (SMM) | $(NH_4)_2SO_4$ | 2.0 g |
| | $KH_2PO_4$ | 6.0 g |
| | $K_2HPO_4$ | 14.0 g |
| | $Na_3Citrate$ | 1.0 g |
| | $MgSO_4$ | 0.2 g |
| | D-Glucose* 50% | 5.0 g |

(continued)

| medium | component | concentration |
|---|---|---|
| | Casaminoacids solution** (1%) | 0.5 g |
| | antibiotics | according to need |
| | $H_2O$, de-ionized | ad 1.0 l |
| | | |
| pre-culture 2 medium | $(NH_4)_2H$-Citrat | 1.00 g/l |
| | $Na_2SO_4$ | 2.00 g/l |
| | $(NH_4)_2SO_4$ | 2.68 g/l |
| | $NH_4Cl$ | 0.50 g/l |
| | $K_2HPO_4$ | 14.60 g/l |
| | Casaminoacid solution (10%) | 0.5 g/l |
| | $NaH_2PO_4xH_2O$ | 4.00 g/l |
| | D-Glucose* (50%) | 5.00 g/l |
| | $MgSO_4$(1 M)* | 1.00 ml/l |
| | TES* (as below) | 3.00 ml/l |
| | | |
| Batch-Medium fermentations (Minimal medium) | $(NH_4)_2H$-Citrat | 1.00 g/l |
| | $Na_2SO_4$ | 2.00 g/l |
| | $(NH_4)_2SO_4$ | 2.68 g/l |
| | $NH_4Cl$ | 0.50 g/l |
| | $K_2HPO_4$ | 14.60 g/l |
| | Casaminoacid solution (10%) | 1 g/l |
| | $NaH_2PO_4xH_2O$ | 4.00 g/l |
| | D-Glucose* (50%) | 25.00 g/l |
| | $MgSO_4$(1 M)* | 1.00 ml/l |
| | TES* (as below) | 3.00 ml/l |
| | | |
| feed medium I | Glucose * $H_2O$ | 654,76 g/l |
| | $MgSO_4$*$7H_2O$ | 23,50 g/l |
| | TES* (as below) | 120 ml/l |
| feed medium II*** | $(NH_4)_2HPO_4$ | 396,00 g/l |
| | | |
| *to be autoclaved separately<br>**separate sterile filtration<br>*** adjusted to pH 7.0 with 85 % v/v H3PO4 before autoclaving | | |

| Trace element solution (TES) | $CaCl_2$x2 $H_2O$ | 0.50 g/l | a |
|---|---|---|---|
| | $FeCl_3$x6 $H_2O$ | 16.70 g/l | a |
| | $Na_2$-EDTA | 20.10 g/l | a |

(continued)

|  | ZnSO$_4$x7 H$_2$O | 0.18 g/l | b |
|---|---|---|---|
|  | MnSO$_4$xH$_2$O | 0.10 g/l | b |
|  | CuSO$_4$x5 H$_2$O | 0.16 g/l | b |
|  | CoCl$_2$x6 H$_2$O | 0.18 g/l | b |
| a: separately pre-solved, and then joined <br> b: weighing in weighing dish, rinsing | | | |

The pH was adjusted with 2 M NaOH and 1 M HCl solution, respectively. For agar plates 15 g/l Euroagar of BD company, were additionally added.

All media were autoclaved at 121 ˚C for about 30 min.

### 5.2 Fermentation with *B.subtilis* TQ356/pMW168.1 (knockout *manP*-mutant).

For fermentation of *B.subtilis* T0356/pMW168.1 the media used for the pre-culture and for the batch were added with 0.05 % and 0.1 % (w/v), respectively, casamino acids (CA).

In a fed-batch-fermentation the cells were grown to high cell density, the promoter was automatically induced upon transition to the fed-phase by glucose limitation.

Fermentation was carried out in a 301 reactor with a batch volume of 8l at the beginning. Depending on OD$_{600}$ a pre-culture were inoculated for adjusting the start OD$_{600}$ to 0.1.

The parameters of the fermentation are set out in the table below:

|  | Batch | Fed-Batch |
|---|---|---|
| T(˚C) | 30, increased to 37 after 11 hours | 37 |
| stirrer speed (rpm) | 380 | regulated depending on oxygen concentration in the medium |
| pressure (bar) | 1,5 | 1,5-1,6 |
| aeration rate | 15l/min | 15-25l/min. |
| pH | 7.0 | 7.0 |
| $\mu$(h$^{-1}$) | 0.34 at the beginning of batch fermentation to 0.21 from 11 hours until Fed-Batch start | actual: 0.09 <br> target: 0.1 |

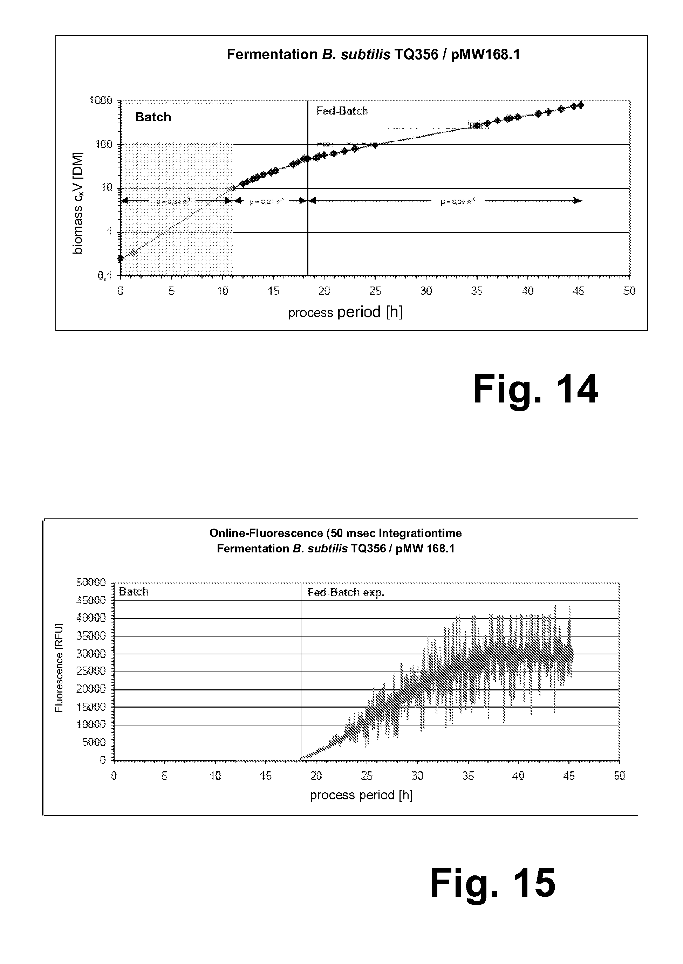

The dry biomass concentration and the monitored fluorescence signal are shown in figures 14 and 15, respectively.

In the figures the concentration of dry biomass cx is plotted logarithmically over the duration of the culture. Batch and fed-batch phase are separated by the perpendicular line.

The monitored fluorescence signal at 535 nm emission wavelength is plotted over the culture period.

As can be seen in figure 15 the fluorescence signal strongly increased after auto-induction on transition from the batch to the fed-batch phase to a maximum of about 30,000 RFU. As can be seen, after 18 h when the glucose concentration was nearly exhausted fluorescence started to increase i.e. after 18 h the expression of GFP (Green Fluorescent Protein) started.

This result clearly shows that by the use of the recombinant bacterial host cell of the present invention without the need of addition of inducer strong expression of the target genes under control of the promoter is obtained.

### 5.3 Fermentation of *B.subtilis* MW343/pMW168.1 (knockout *manA*-mutant).

For comparison a similar fermentation as set out in 5.1 above was run with *B.subtilis* MW343/pMW168.1 with deleted *manA* in the mannose operon.

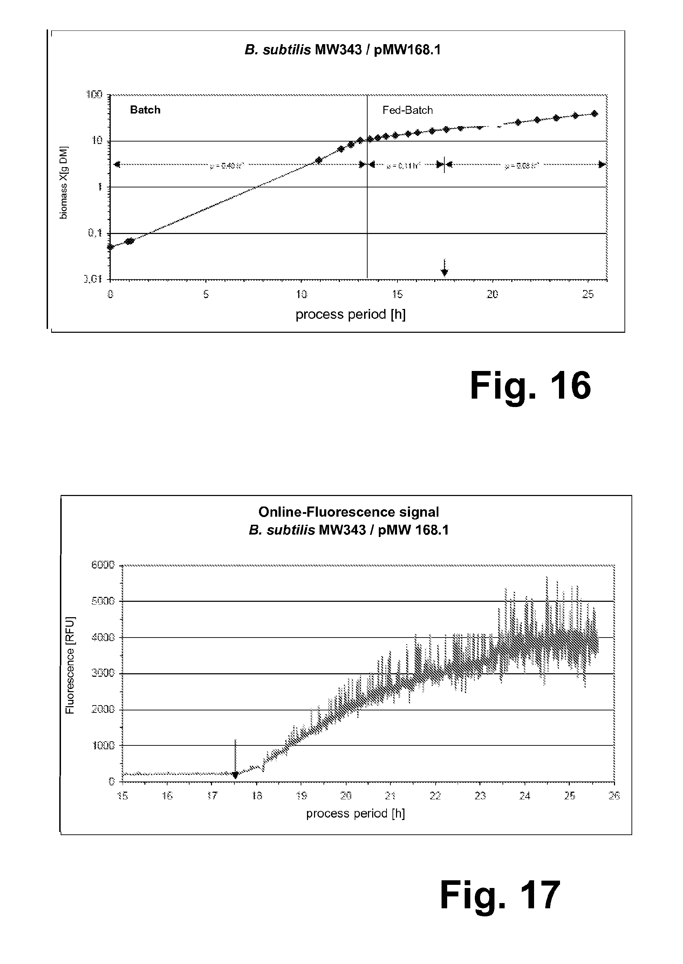

The cells were grown until about 40 OD$_{600}$ and, then, induced via impact induction with single addition of 2.5 g/l mannose after about 17.5 hours of cultivation (indicated in figs. 16 and 17 by an arrow).

| The parameters of the fermentation are shown in the table below: | Batch | Fed-Batch |
|---|---|---|
| T (˚C) | 30 | 37 |
| stirrer speed (rpm) | 400 | regulated depending on oxygen concentration in the medium |
| pressure | 1.5 | 1.5 |
| aeration rate (l/min) | 2 | 2 |
| pH | 7.0 | 7.0 |
| $\mu$ ($h^{-1}$) | 0.40 | 0.11 (until induction)<br>0.08 (upon induction) |

The dry biomass concentration and the monitored fluorescence signal are shown in figures 16 and 17, respectively. In figure 16 the concentration of the dry biomass $c_x$ is plotted logarithmically over duration of the culture. Batch and fed-batch phase are separated by the perpendicular line.

The monitored fluorescence signal at 535 nm emission wavelength is plotted over the culture period. An arrow indicate the point of induction.

As shown in figure 17 the fluorescence signal strongly increased after addition of D-mannose up to a maximum of about 4,000 RFU within the first five hours after induction. Though considerable reduction of inducer is obtained, compared to the fermentation of the knockout *manP B.subtilis* TQ356 the fluorescence signal is, however, significantly decreased.

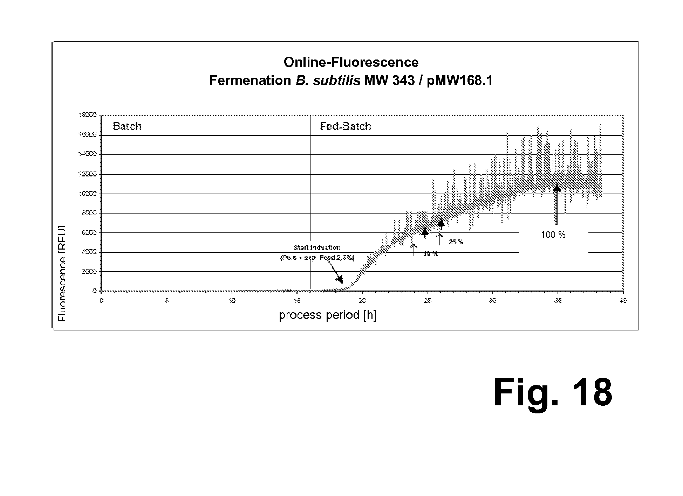

A further fermentation was run with *B.subtilis* MW343/pMW168.1 with an additional exponential supplementary feeding of mannose for maintaining the mannose level in the culture medium at about 2 g/l, with a total of mannose feed of 100 g (fed of mannose in % is indicated in Fig. 18 by arrows).

As shown in figure 18 the fluorescence signal is increased to about 11,000 RFU compared to the first run, though still lower than the signal obtained with the knockout *manP B.subtilis* TQ343. These results show that also by deletion of the gene encoding *manA* the amount of mannose necessary for induction can be reduced.

## Claims

1. Recombinant bacterial host cell, wherein the recombinant bacterial host cell is capable of utilizing more than one carbon source, whose catabolism is subject to carbon catabolite repression and phosphoenol pyruvate: carbohydrate phosphortransferase system, wherein the bacterial host cell is genetically altered to be incapable of deactivation of the transcriptional regulator protein specific for a carbon source inducible promoter in the absence of the inducing carbon source, wherein the inducing carbon source is a secondary carbon source for the bacterial host cell.

2. Recombinant bacterial host cell according to claim 1 further comprising a vector, wherein the vector comprises a heterologous nucleic acid sequence encoding a polypeptide operably linked to a carbon source inducible promoter, wherein the promoter of the vector is regulated by the transcriptional regulator protein for which the bacterial host cell is genetically altered to be incapable of deactivation, wherein the inducing carbon source of the promoter of the vector is the secondary carbon source specific for the transcriptional regulator protein.

3. Recombinant bacterial host cell according to claims 1 or 2, wherein by genetical alteration of the bacterial host cell deactivation of the transcriptional regulator protein by phosphorylation by the enzyme EII, specific for said transcriptional regulator protein, is prevented.

4. Recombinant bacterial host cell according to any of the claims 1 to 3, wherein in the genome of the bacterial host cell the gene is deleted which encodes for the phosphoryl group transferring enzyme EII specific for the transcriptional regulator protein.

5. Recombinant bacterial host cell according to any of the claims 1 to 3, wherein in the genome of the bacterial host cell the gene encoding for the transcriptional regulator protein is genetically altered so that the transcriptional regulator protein expressed by said gene is incapable of binding a phosphoryl group transferred from enzyme EII.

6. Recombinant bacterial host cell according to claim 5, wherein into the vector is integrated the genetically manipulated gene of the bacterial host cell encoding for the transcriptional regulator protein incapable of binding a phosphoryl group transferred from the corresponding enzyme EII.

7. Recombinant bacterial host cell according to any of the claims 1 to 6, wherein the bacterial host cell is selected from the phylum Firmicutes and of the order enterobacteriales.

8. Recombinant bacterial host cell according to claim 7, wherein the bacterial host cell is selected from Bacilli, Clostridia and Enterobacteriales.

9. Recombinant bacterial host cell according to any of the claim 1 to 8, wherein the promoter is the promoter of a catabolic operon for the catabolism of a carbon source subject to PTS.

10. Recombinant prokaryotic host cell according to claim 9, wherein the transcriptional regulator protein is an antiterminator or activator of said promoter.

11. Method for producing a heterologous polypeptide in a recombinant host cell comprising the steps of growing a bacterial host cell according to any of the claims 2 to 10 in a cell culture medium under suitable conditions for allowing expression of said polypeptide and recovering the polypeptide from the cells or from the cell culture, wherein the medium comprises at least one carbon source which is not an inducer for said promoter but stimulates repression of the promoter by carbon catabolite repression, and wherein expression of the heterologous polypeptide under control of said promoter is induced by allowing the concentration of said at least one carbon source to decrease under a level necessary for carbon catabolite repression.

12. Method for producing a heterologous polypeptide in a bacterial host cell according to claim 11, wherein the culture medium comprises a pre-determined amount of said at least one carbon source, but no inducing secondary carbon source.

13. Method for producing a heterologous polypeptide in a bacterial host cell according to claim 11 or 12, wherein the culture medium comprises casamino acids.

14. Method according to any of claims 11 to 13, wherein during expression of the heterologous polypeptide the growth rate is adjusted to be 0.2 $\mu$ or less.

15. Use of a bacterial host cell according to any of the claims 1 to 10 for the regulated expression of a heterologous nucleic acid sequence encoding a polypeptide in the bacterial host cell.

## Operon-structure (Mannose)

# Fig. 1

## Mannose-Catabolism

# Fig. 2

# Fig. 3

pSUN291

pSUN385.2

TGAATTTCTGCTGAATATACATTACATAGCAAACTCAAAGAGTATAAAAA

pSUN386.9

*Hind*III                -35

TCGCTTTTTTCCGG**AAGCTT**CGGTAAAAAACGAAA***CTTTTG***TCTCTATGA

IRI-R               IRI-R

-10            +1

TTTTGTTT***TATAAT***GTAAACG***G***TTTCTTATATAGTATACTTATACTTATC

*cre*

RBS

AATTTGCTCAAGTAGATACTGACA**GGAAGG**ATAGAAAAACAG**ATG**

*manR*

# Fig. 4

pSUN284.1

*NruI*

TCGCGATTGCCAC—170bp—GTCATGACATTTTTATCTCATTTG

............................................... *manR* ...............................................

pSUN290

GATTAT**TAA**AAGCAGGGATTATTCCTTGCTTTTTTTGTTATAGG

*manR* ·······▶|

pSUN297.5

GAAAAATGCCTTTATTACCGGAACCTATGGTAAAAAAAGCG ***ATT***
　　　　　　　　　　　　　　▶　◀
　　　　　　　　　IRI-P　　　　　　　IRI-P

-35　　　　　　　　　　　-10　　　　　+1 *BglII*

***TTA***ATGAGCTGATTTCGGTA***TACAGT***TGAGAC***A***AGATCTTATTA

　　　　*XbaI*　　　　　　*AflII*　　　　　　*NdeI*

TTCACACTTTCTAGAAATAATTTTCTTAAGAAGGAGATATACATATG

　　　　　　　　　　　　　　　　　　*lacZ* ▶

# Fig. 5

Fig. 6

Fig. 7

# Fig. 8

# Fig. 9

## Fig. 10

## Fig. 11

**Fig. 12**

**Fig. 13**

Fermentation *B. subtilis* TQ356 / pMW168.1

**Fig. 14**

Online-Fluorescence (50 msec Integrationtime
Fermentation *B. subtilis* TQ356 / pMW 168.1

**Fig. 15**

**B. subtilis MW343 / pMW168.1**

Fig. 16

**Online-Fluorescence signal**
**B. subtilis MW343 / pMW 168.1**

Fig. 17

**Online-Fluorescence**
**Fermenation *B. subtilis* MW 343 / pMW168.1**

Fig. 18

Europäisches
Patentamt
European
Patent Office
Office européen
des brevets

**EUROPEAN SEARCH REPORT**

Application Number

EP 10 17 1252

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TIANKI SUN: "Regulation des Mannose-Operons in Bacillus subtilis, Dissertation zur Erlangung der Würde eines Doktors in Naturwissenschaften", INTERNET CITATION, 16 April 2010 (2010-04-16), pages I-VI, XP002604428, Retrieved from the Internet: URL:http://elib.uni-stuttgart.de/opus/voll texte/2010/5249/pdf/Regulation_ des_Mannose_Operons.pdf [retrieved on 2010-11-10] | 1-10 | INV. C12N15/63 C12N15/75 |
| Y | * the whole document, in particular p.3-4, 82, 86-88, 123-127 * ----- | 11-15 | |
| X | SUN TIANQI ET AL: "Characterization of a mannose utilization system in Bacillus subtilis", JOURNAL OF BACTERIOLOGY, AMERICAN SOCIETY FOR MICROBIOLOGY, WASHINGTON, DC; US, vol. 192, no. 8, 1 April 2010 (2010-04-01) , pages 2128-2139, XP002604427, ISSN: 0021-9193, DOI: DOI:10.1128/JB.01673-09 [retrieved on 2010-02-05] | 1-10 | TECHNICAL FIELDS SEARCHED (IPC) C12N |
| Y | * the whole document * ----- -/-- | 11-15 | |

The present search report has been drawn up for all claims

| Place of search | Date of completion of the search | Examiner |
|---|---|---|
| Munich | 17 December 2010 | Bassias, Ioannis |

EPO FORM 1503 03.82 (P04C01)

**Europäisches Patentamt**
**European Patent Office**
**Office européen des brevets**

# EUROPEAN SEARCH REPORT

Application Number

EP 10 17 1252

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOBISCH STEFFEN ET AL: "Regulation of the lic operon of Bacillus subtilis and characterization of potential phosphorylation sites of the LicR regulator protein by site-directed mutagenesis", JOURNAL OF BACTERIOLOGY, vol. 181, no. 16, August 1999 (1999-08), pages 4995-5003, XP002614551, ISSN: 0021-9193 | 1-10 | |
| Y | * the whole document, in particular Fig. 4, Table 4 and p. 5000 right-hand column * ----- | 11-15 | |
| Y | GÖRKE BORIS ET AL: "Carbon catabolite repression in bacteria: many ways to make the most out of nutrients.", NATURE REVIEWS. MICROBIOLOGY AUG 2008 LNKD- PUBMED:18628769, vol. 6, no. 8, August 2008 (2008-08), pages 613-624, XP009142600, ISSN: 1740-1534 * the whole document * ----- | 11-15 | |
| Y | DEUTSCHER ET AL: "The mechanisms of carbon catabolite repression in bacteria", CURRENT OPINION IN MICROBIOLOGY, CURRENT BIOLOGY LTD, GB, vol. 11, no. 2, 1 April 2008 (2008-04-01), pages 87-93, XP022616728, ISSN: 1369-5274, DOI: DOI:10.1016/J.MIB.2008.02.007 [retrieved on 2008-03-21] * the whole document * ----- | 11-15 | |

TECHNICAL FIELDS SEARCHED (IPC)

-/--

The present search report has been drawn up for all claims

| Place of search | Date of completion of the search | Examiner |
|---|---|---|
| Munich | 17 December 2010 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 17 1252

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ABRANCHES JACQUELINE ET AL: "Characterization of Streptococcus mutans strains deficient in EIIAB Man of the sugar phosphotransferase system", APPLIED AND ENVIRONMENTAL MICROBIOLOGY, AMERICAN SOCIETY FOR MICROBIOLOGY, US, vol. 69, no. 8, 1 August 2003 (2003-08-01), pages 4760-4769, XP002558891, ISSN: 0099-2240, DOI: DOI:10.1128/AEM.69.8.4760-4769.2003 ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 17 December 2010 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006133210 A2

**Non-patent literature cited in the description**

- **MANIATISE et al.** Molecular Cloning A laboratory Manual. Cold Spring Harbor Laboratory, 1982
- **AUSUBEL et al.** Current protocols in molecular biology. John Wiley and Sohns, 1984
- **J. STÜLKE et al.** Regulation of carbon catabolism in Bacillus species. *Annu. Rev. Microbiol.,* 2000, vol. 54, 849-880
- **GÖRKE B. ; STÜLKE J.** Carbon catabolite repression in bacteria: many ways to make the most out of nutrients. *Nat. Rev. Microbiol.,* 2008, vol. 6, 613-624
- **GOSSET G. et al.** Transcriptome analysis of Crp-dependent catabolite control of gene expression in Escherichia coli. *J. Bacteriol.,* 2004, vol. 186, 3516-3524
- **MARTINEZ-ANTONIO A. et al.** Identifying global regulators in transcriptional regulatory networks in bacteria. *Curr. Opin. Microbiol.,* 2003, vol. 6, 482-489
- **KUNST F. N. et al.** The complete genome sequence of gram-positive bacterium B.subtilis. *Nature,* 1997, vol. 390, 249-256
- **RÖDER D. L. et al.** Marker-exchange mutagenesis of a pektate lyase isozyme gene in Erwinia chrys anthemy. *J. Bacteriol.,* 1985, vol. 164 (1), 51-56
- **WELLS J. A. et al.** Cassette mutagenesis: an efficient method for generation of multiple mutations at defined sites. *Gene,* 1985, vol. 34 (2 - 3), 315-323
- **KORTZ et al.** *J. Biotechnol.,* 1995, vol. 39, 59-65
- **KULLA.** *Arch. Microbiol.,* 1983, vol. 135, 1
- **BERTANI et al.** *J. Bacteriol.,* 1951, vol. 62, 293-300

- **WILMS et al.** *Biotechnol. Biong.,* 2001, vol. 73, 95-103
- **YANISCH-PERRON C. et al.** *Gene,* 1985, vol. 33, 103-119
- **MICHEL J. F. et al.** *J. Appl. Bacteriol.,* 1970, vol. 33, 220-227
- **LURIA S. E. et al.** *Virology,* 1960, vol. 12, 348-390
- **MARTIN-VERSTRAETE I. et al.** *J. Mol.Biol.,* 1990, vol. 214, 657-671
- **Chung C.T. et al.** *Proc. Natl. Acad. Sci. USA,* 1989, vol. 86, 2172-2175
- **Harwood C.R.** Molecular Biological Methods for Bacillus. John Wiley & Sons Ltd, 1990
- **Miller J. H.** experiments in molecular genetics. Cold Spring Harbor, 1972
- **Altenbuchner et al.** *Methods Enzymol.,* 1992, vol. 216, 457-466
- **MacKenzie et al.** *Plasmid,* 1986, vol. 15, 93-103
- **Recsai et al.** *Proc. Natl. Acad. Sci. USA,* 1987, vol. 84, 1127-1131
- **Haldimann A. et al.** *J. Bacteriol.,* 2001, vol. 183, 6384-6393
- **Wegerer et al.** *BMC. Biotechnol.,* 2008, vol. 8, 2
- **Geurout-Fleury et al.** *Gene,* 1996, vol. 180, 57-61
- **Lagodich et al.** *Mol. Biol. (Mosk),* 2005, vol. 39, 345-348
- **ANAGNOSTOPULOS et al.** Requirements for transformation in Bacillus subtilis. *J. Bacteriol.,* 1961, vol. 81, 741-746
- **WILMS et al.** *Biotechnol. Bioeng.,* 2001, vol. 73, 95-103

SEQUENCE LISTING

<110> Lonza AG

<120> Regulation of inducible promoters

<130> 9000-9305

<160> 5

<170> PatentIn version 3.3

<210> 1
<211> 143
<212> DNA
<213> Bacillus subtilis

<400> 1
tagggaaaaa tgcctttatt accggaacct atggtaaaaa aagcgatttt aatgagctga    60

tttcggtata cagttgagac aagatcttat tattcacact ttctagaaat aattttctta    120

agaaggagat atacatatga cac    143


<210> 2
<211> 80
<212> DNA
<213> Bacillus subtilis

<400> 2
tagggaaaaa tgcctttatt accggaacct atggtaaaaa aagcgatttt aatgagctga    60

tttcggtata cagttgagac    80


<210> 3
<211> 195
<212> DNA
<213> Bacillus subtilis

<400> 3
tgaatttctg ctgaatatac attacatagc aaactcaaag agtataaaaa tcgctttttt    60

ccggaagctt cggtaaaaaa cgaaactttt gtctctatga ttttgtttta taatgtaaac    120

ggtttcttat atagtatact tatactatca atttgctcaa gtagatactg acaggcttaa    180

gaaggagata tacat    195


<210> 4
<211> 128
<212> DNA
<213> Bacillus subtilis

<400> 4
tgaatttctg ctgaatatac attacatagc aaactcaaag agtataaaaa tcgctttttt    60

ccggaagctt cggtaaaaaa cgaaactttt gtctctatga ttttgtttta taatgtaaac    120

ggtttctt    128


<210> 5
<211> 121
<212> DNA
<213> Bacillus subtilis

```
<400>  5
tgaatttctg ctgaatatac attacatagc aaactcaaag agtataaaaa tcgctttttt      60

ccggaagctt cggtaaaaaa cgaaactttt gtctctatga ttttgtttta taatgtaaac     120

g                                                                     121
```